# EUROPEAN PATENT APPLICATION

(11) **EP 2 918 278 A1**
(43) Date of publication of application: **16.09.2015**
(21) Application number: 14382090.0
(22) Date of filing: 14.03.2014
(51) Int. Cl.: A61K 36/03, A61K 36/04, A61P 29/00, A61K 8/00

(54) **Algal extracts comprising fucoxanthin and fucoxanthinol**

(71) Applicant: Greenaltech S.L., 08028 Barcelona (ES)
(72) Inventor: Ortiz Almirall, Xavier, E-08028 Barcelona (ES); Tourino Eirin, Sonia, E-08028 Barcelona (ES); Álvarez Mico, Xavier, E-08028 Barcelona (ES); Durany Turk, Olga, E-08028 Barcelona (ES)
(74) Representative: ABG Patentes, S.L.

(57) **Abstract**

The invention relates to an algal extract comprising fucoxanthin and fucoxanthinol, its production process, and the use thereof in cosmetic, cosmeceutical, food, nutraceutical, or pharmaceutical applications.

## Description

### FIELD OF THE INVENTION

The invention relates to an algal extract comprising fucoxanthin and fucoxanthinol, its production process, and the use thereof in cosmetic, cosmeceutical, food, nutraceutical, or pharmaceutical applications.

### BACKGROUND OF THE INVENTION

Inflammation forms part of the complex biological response of vascular tissues to harmful stimuli, such as pathogens, damaged cells, or irritants. The classical signs of acute inflammation are pain, heat, redness, swelling, and loss of function. Inflammation is a protective attempt by the organism to remove the injurious stimuli and to initiate the healing process. Inflammation is considered as a mechanism of innate immunity. Inflammation can be classified as either acute or chronic. Acute inflammation is the initial response of the body to harmful stimuli and is achieved by the increased movement of plasma and leukocytes (especially granulocytes) from the blood into the injured tissues. Chronic inflammation is involved in multiple diseases, such as periodontal disease, colitis, arthritis, atherosclerosis, Alzheimer's, asthma, multiple sclerosis, and inflammatory bowel diseases.

A cascade of biochemical events propagates and matures the inflammatory response, involving the local vascular system, the immune system, and various cells within the injured tissue. Prolonged inflammation, known as chronic inflammation, leads to a progressive shift in the type of cells present at the site of inflammation and is characterized by simultaneous destruction and healing of the tissue from the inflammatory process.

Inflammatory abnormalities are a large group of disorders that underlie a vast variety of diseases. The immune system is often involved with inflammatory disorders, demonstrated in both allergic reactions and some myopathies, with many immune system disorders resulting in abnormal inflammation. Non-immune diseases with etiological origins in inflammatory processes include cancer, atherosclerosis and ischaemic heart disease. Examples of disorders associated with inflammation include acne vulgaris, asthma, autoimmune diseases, celiac disease, chronic prostatitis, glomerulonephritis, hypersensitivities, inflammatory bowel disease, pelvic inflammatory disease, reperfusion injury, rheumatoid arthritis, sarcoidosis, or transplant rejection. Further, an inflammatory component is also present in atherosclerosis, allergies, myopathies, and cancer.

There are believed to be nearly 860 million metabolic syndrome patients in six major countries in the world. The number of obese people is increasing in Japan due to more Westernized and irregular dietary habits and lack of exercise resulting from a more convenient lifestyle. According to a trial calculation of the Ministry of Health, Labour and Welfare, approximately 190 million people (aged between 40 and 70) either have metabolic syndrome or will so in the future, which is one out of two men and one out of five women in the age group. Although obesity, high blood pressure, hyperlipidemia, and diabetes were formerly considered to be independent lifestyle diseases, accumulated visceral fat must also be considered as a common cause of them. Accumulated visceral fat increases free fatty acid in the blood and triggers hyperlipidemia and insulin resistance. Various physiologically active substances, namely adipocytokines, are secreted from visceral fat tissues. Excessively accumulated visceral fat has been confirmed to destroy the secretion balance or these substances and cause metabolic syndrome. White adipose tissues and brown adipose tissues are found in human fat tissues and perform different functions. White adipose tissues store excessive calories as fat. Increased white adipose tissues signify obesity. On the contrary, brown adipose tissues maintain body temperature at a certain level and consume excessive calories by degrading fat and generating heat. These activities are performed by uncoupling protein 1 (UCP1) existing selectively in mitochondrial inner membrane of brown adipose tissues. Various biogenic factors are involved in the expression of UCP1. Food components capsaicin, capsiate, and caffeine increase the UCP1 expression by increasing the secretion of noradrenaline and EPA and DHA do the same by becoming PPARγ ligand. However, in human bodies, the amount of brown adipose tissues decreases with age so the increase of brown adipose tissues does not necessarily contribute to the prevention of obesity. Therefore, the expression of UCP1 in white adipose tissues is desired so that UCP1 can accelerate the oxidation of white fat and conversion of energy to heat and in turn decrease white adipose tissues.

A large variety of proteins are involved in inflammation, and any one of them is open to a genetic mutation which impairs or otherwise dysregulates the normal function and expression of that protein, being interleukin 8 (IL-8), also known as CXCL8, one of the relevant cell-derived mediators of the inflammatory process.

IL-8 is a chemokine produced by macrophages and other cell types such as epithelial cells, airway smooth muscle cells and endothelial cells.IL-8 has two primary functions. It induces chemotaxis in target cells [neutrophil granulocytes are the primary target cells of IL-8, although there are a relatively wide range of cells (endothelial cells, macrophages, mast cells and keratinocytes) that respond to this chemokine], causing them to migrate toward the site of infection. IL-8 also induces phagocytosis once they have arrived. IL-8 is also known to be a potent promoter of angiogenesis.

IL-8 is often associated with inflammation; in fact, IL-8 has been cited as a proinflammatory mediator in gingivitis and psoriasis. Further, it is believed that IL-8 plays a role in the pathogenesis of bronchiolitis, a common respiratory tract disease caused by viral infection.

There are currently many anti-inflammatory/anti-pain agents that are used to treat inflammation and pain in patients. These agents generally include steroids and non-steroidal anti-inflammatory drugs (NSAIDS). Steroids generally act to reduce inflammation by binding to the glucocorticoid receptor, whereas NSAIDS generally act to inhibit both cyclooxygenase-1 (COX-1) and cyclooxygenase (COX-2), thus inhibiting the catalysis of the formation of the inflammation messengers prostaglandins and thromboxane.

These anti-inflammatory/anti-pain agents are widely used but can have many adverse side effects. Steroids have been shown to cause hyperglycemia, insulin resistance, diabetes, osteoporosis, cataracts, anxiety, depression, colitis, hypertension, ictus, erectile dysfunction, hypogonadism, hypothyroidism, amenorrhea, retinopathy, and teratogenic defects. NSAIDS have been shown to cause gastrointestinal adverse reactions (nausea, dyspepsia, gastric ulceration and bleeding, diarrhea), myocardial infarction, stroke, erectile dysfunction, renal adverse reactions (salt and fluid retention, hypertension, interstitial nephritis, nephrotic syndrome, acute renal failure, acute tubular necrosis), photosensitivity, teratogenic defects, premature birth, miscarriage, raised liver enzymes, headache, dizziness, hyperalaemia, confusion, bronchospasm, rashes, swelling, and irritable bowel syndrome.

Therefore, there remains a need for an anti-inflammatory agent that is effective in treating inflammation but reduces the risk of the above adverse reactions.

An option to satisfy that need comprises finding compounds from nature, which unlike the steroidal and non-steroidal anti-inflammatory agents have less, or preferably, no side effect, and yet which exhibit substantially equal anti-inflammatory action to the above anti-inflammatory agents. With regard to components derived from natural substances having an anti-inflammatory action, it has been reported that extracts from various plants such as an extract of bark of Yamamomo (*Myrica rubra*) exhibit a hexosaminidase release-inhibitory activity and extracts from leaves of *Camellia japonica L., Camellia japonica* L. cv. or *Camellia sasanqua* T. have a potent anti-inflammatory action.

Nevertheless, the demand for finding compounds from nature, which unlike the steroidal and non-steroidal anti-inflammatory agents, have no side effect such as hormone action and cause no enteric disorders, and yet which exhibit equal anti-inflammatory action to the above anti-inflammatory agents is still necessary. It is an objective of the present invention to provide such substances and anti-inflammatory agents utilizing them.

Inventors have now found that an algal extract comprising fucoxanthin and fucoxanthinol exerts an unexpectedly high anti-inflammatory activity. It has been reported that fucoxanthin promotes fat burning within fat cells in white adipose tissue and has strong anticancer activity, whereas fucoxanthinol exhibits suppressive effects on lipid accumulation during adipocyte differentiation.

### BRIEF DESCRIPTION OF THE DRAWINGS

Figure 1 shows an overlaid HPLC chromatogram of the initial fucoxanthin, C-MEDPA [chemical derivatization method (Example 1)] and E-MEDPA [enzymatic derivatization method (Example 1)] reaction products.
Figure 2 shows an overlaid HPLC chromatogram of a *Pavlova lutheri* extract before and after the C-MEDPA derivatization process.
Figure 3 shows the HPLC chromatogram of an *Isochrysis galbana* extract before the C-MEDPA derivatization process (A) and after the C-MEDPA derivatization process (B).
Figure 4 shows the HPLC chromatogram of an *I. galbana* extract before the enzymatic E-MEDPA derivatization process (A) and after the E-MEDPA derivatization process with cholesterol esterase (B).

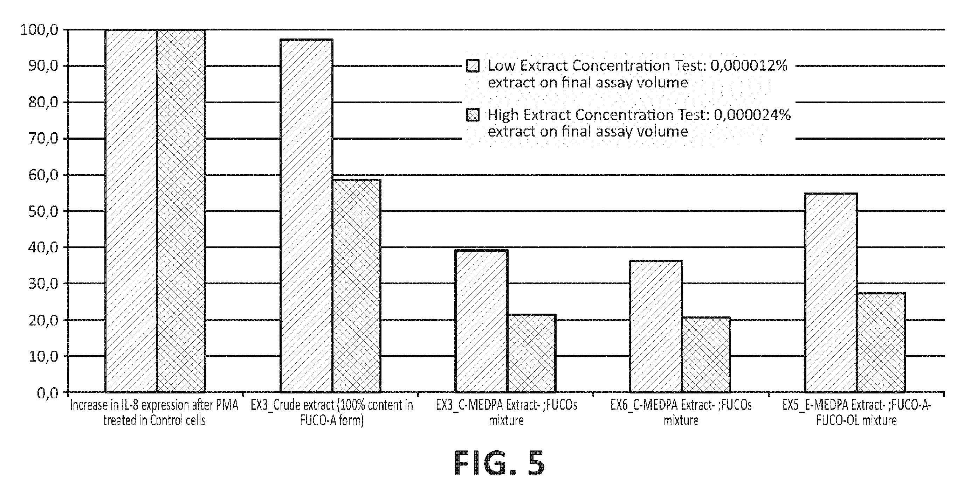

Figure 5 is a bar diagram showing the normalized results of the production of IL-8 by keratinocytes treated first (after 2 days of growth) with the extracts under examination and second (24 h later) challenged with PMA for 5 hours previous growth. Results are normalized to reduction in IL-8 production related to keratinocytes untrated after 2 days of growth and equally challenged with PMA (Untrated Control). Extract Treated Samples before PMA challenge: *I. galbana* extract containing fucoxanthin [EX_3 Crude extract 100% content in FUCO-A form)] and with extracts, at two concentrations, comprising fucoxanthin and related products (fucoxanthin and fucoxanthinol, and, optionally amarouciaxanthin A and/or isofucoxanthiol) obtained by subjecting *I. galbana* crude extracts to treatment with the C-MEDPA derivatization process or with the E-MEDPA derivatization process [Extracts: (i) EX3_C-MEDPA Extract-; FUCOs mixture; (ii) EX6_C-MEDPA Extract-; FUCOs mixture; and (iii) EX5_E-MEDPA Extract; FUCO-A-FUCO-OL mixture].
Figure 6 is a bar diagram showing the results of the relative lipolytic activity (%) of an algal extract ("Extract -1A"), at two different concentrations, obtained from *Thalassiosira pseudonana* after treatment with the C-MEDPA derivatization process.
Figure 7 shows GC chromatograms of the *I. galbana* extracts obtained after treatment with the C-MEDPA or E-MEDPA derivatization process in Examples 2, 3 and 5.

### DETAILED DESCRIPTION OF THE INVENTION

The inventors of the instant invention have found that an algal extract comprising fucoxanthin and fucoxanthinol together with other algal components, such as, for example, amarouciaxanthin A and/or isofucoxanthinol, among others, exerts an unexpectedly high anti-inflammatory activity, as it is shown in Example 9, wherein the production of the pro-inflammatory chemokine mediator, interleuquin 8 (IL-8), is strongly repressed in keratinocytes treated with said algal extract and challenged with PMA (phorbol-12-myristate-13-acetate). Said algal extract is produced by subjecting the biomass, preferably, the complete biomass, obtained from a culture of fucoxanthin producing algae to a chemical or enzymatic derivatization process on fucoxanthin without the need of isolating and purifying fucoxanthin from the production medium.

Thus, the present invention relates to an algal extract comprising fucoxanthin and fucoxanthinol together with other algal components, its production process and its applications. Some applications relate to its use as anti-inflammatory agent whereas some other applications relate to the uses of the components thereof; by illustrative, it is well-known that fucoxanthin and fucoxanthinol can be used as anti-obesity agents since they have a fat burner effect, or as antiangiogenic agents, etc. Thus, said algal extract can be used in a lot of cosmetic, cosmeceutical, food, nutraceutical, or pharmaceutical applications.

### Algal extract of the invention

In an aspect, the invention relates to an algal extract, hereinafter referred to as the "algal extract of the invention", comprising fucoxanthin and fucoxanthinol, and, optionally, amarouciaxanthin A and/or isofucoxanthinol, together with other algal components.

As used herein, the term "algal extract" refers to a product obtained from algae, for example, by subjecting an algae culture to specific treatments. The components present in an algal extract will vary depending on the algae and the treatments applied thereon.

As used herein, the term "fucoxanthin" refers to the acetic acid [(1S,3R)-3-hydroxy-4-[(3E,5E, 7E,9E, 11E, 13E, 15E)-18-[(1S,4S,6R)-4-hydroxy-2,2,6-trimethyl-7-oxabicyclo [4.1.0]heptan-1-yl]-3,7,12,16-tetramethyl-17-oxooctadeca-1,3,5,7,9,11,13,15-octaenylidene]-3,5,5-trimethylcyclohexyl]ester] compound of formula

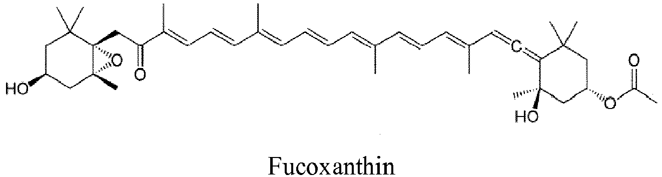

Fucoxanthin is a xanthophyll that can be found as an accessory pigment in the chloroplasts of a great number of algae, for example, brown algae and most heterokonts, giving them a brown or olive-green color. Some metabolic and nutritional studies carried out on rats and mice indicate that fucoxanthin promotes fat burning within fat cells in white adipose tissue by increasing the expression of thermogenin. Further, it has been reported to have strong anticancer activity [Masashi Hosokawa et al., Biochimica et Biophysica Acta, 1675, pp. 113-119 (2004)]. Additional uses of fucoxanthin include its use in, for example, treatment of skin pigmentation, metabolic syndrome management, medical treatment and prevention of virus associated malignancy such as adult T-cell leukemia and Burkitt lymphoma, treatment of hyperuricemia, osteoporosis and depression, as well as anticancer agent, anti-neoplastic agent, neovascularization inhibitor, adiponectin production accelerator, cholesterol lowering agent, antihypertensive agent or antiallergic agent.

Fucoxanthin can be present in the algal extract of the invention in a very broad concentration range. Nevertheless, in a particular embodiment, fucoxanthin is present in the algal extract of the invention at a concentration comprised between about 0.001 % and about 90% by weight with respect to the total weight of the algal extract of the invention, preferably between 0.01% and 75% by weight, more preferably between 0.1% and 45% by weight, still more preferably between 1% and 15% by weight. In some specific embodiments, the algal extract of the invention comprises 1% to 15% by weight of fucoxanthin, such as, for example, between 1% and 10% by weight, between 2% and 4% by weight or between 5 % and 10% by weight.

As used herein, the term "fucoxanthinol" refers to the deacetylated form of fucoxanthin, i.e., a substance in that the -OCOCH3 group of fucoxanthin is substituted by a -OH group, of formula

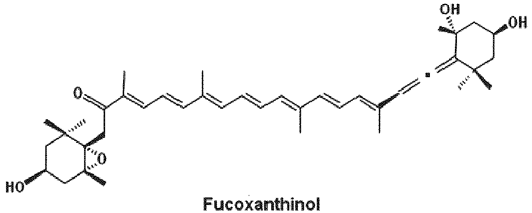

Fucoxanthin is hydrolyzed into fucoxanthinol in the gastrointestinal tract before absorption in the intestine. Fucoxanthinol can be produced from isolated fucoxanthin by enzymatic hydrolysis with a porcine pancreas lipase [WO 2007060811 A1].

Fucoxanthinol exhibits suppressive effects on lipid accumulation during adipocyte differentiation. The suppressive effect of fucoxanthinol is stronger than that of fucoxanthin. Additional uses of fucoxanthinol include treatment of osteosarcoma, skin pigmentation, as well as anticancer agent or anti-neoplastic agent.

Fucoxanthinol can be present in the algal extract of the invention in a very broad concentration range. Nevertheless, in a particular embodiment, fucoxanthinol is present in the algal extract of the invention at a concentration comprised between about 0.001 % and about 90% by weight with respect to the total weight of the algal extract of the invention, preferably between 0.01% and 75% by weight, more preferably between 0.1% and 45% by weight, still more preferably between 1% and 15% by weight. In some specific embodiments, the algal extract of the invention comprises 1% to 15% by weight of fucoxanthinol, such as, for example, between 1% and 10% by weight, between 2% and 4% by weight or between 5% and 10% by weight.

As used herein, the term "amarouciaxanthin A" refers to the (3'S,5'R,6S,6'R,8'R)-3',5',6-trihydroxy-4,7'-didehydro-5',6,7,8-tetrahydro-β,β-carotene-3,8-dione of formula

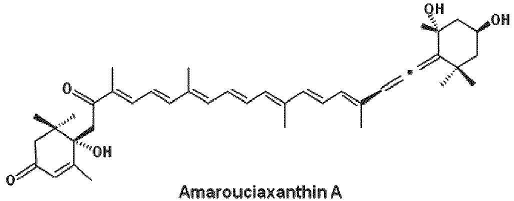

Amarouciaxanthin A can be separated, for example, from acetone extracts from *Amaroucium pliciferum* with silica gel column chromatography using acetone/n-hexane (1:9, v/v) [Mi-Jin et al., J. Agric. Food Chem. 2011, 59, 1646-1652].

Amarouciaxanthin A can be used as an agent to supress adipocyte differentiation on 3T3-L1 cells (Yim et al., J. Agric. Food Chem. 2011 Mar 9;59(5):1646-52).

Amarouciaxanthin A can be present, or not, in the algal extract of the invention. Thus, in a particular embodiment, the algal extract of the invention does not contain amarouciaxanthin A. Alternatively, if present, the concentration of amarouciaxanthin A in the algal extract of the invention can vary broadly. Thus, in a particular embodiment, the algal extract of the invention contains amarouciaxanthin A in a concentration between more than 0% by weight and about 90% by weight with respect to the total weight of the algal extract of the invention, preferably between about 0.001% and about 80% by weight, more preferably between 0.01% and 70% by weight, still more preferably between 0.1 % and 50% by weight, even still more preferably between 1% and 15% by weight. In some specific embodiments, the algal extract of the invention comprises 1% to 15% by weight of amarouciaxanthin A, such as, for example, between 0.02% and 5% by weight, or between 0.6% and 4% by weight.

As used herein, the term "isofucoxanthinol" refers to the (3S,5R,3'S,5'R,6'R)-3',5'3',6'-tetrahydroxy-6',7'-didehydro-5,8,5',6'-tetrahydro-β,β-carotene-8-one of formula

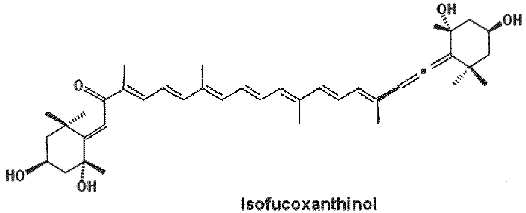

Isofucoxanthinol can be prepared from fucoxanthinol by alkaline treatment with 1% potassium hydroxide (KOH) for 35 min at room temperature [Mi-Jin et al., J. Agric. Food Chem. 2011, 59, 1646-1652].

Isofucoxanthinol can be present, or not, in the algal extract of the invention. Thus, in a particular embodiment, the algal extract of the invention does not contain isofucoxanthinol. Alternatively, if present, the concentration of isofucoxanthinol in the algal extract of the invention can vary broadly. Thus, in a particular embodiment, the algal extract of the invention contains isofucoxanthinol in a concentration between more than 0% by weight and about 10% by weight with respect to the total weight of the algal extract of the invention, preferably between about 0.001% and about 8% by weight, more preferably between 0.01 % and 5% by weight, still more preferably between 0.1 % and 1.5% by weight. In some specific embodiments, the algal extract of the invention comprises between 0.02% and 5% by weight, or between 0.1% and 1.5% by weight. The algal extract of the invention may also contain other algal components. As it is used herein, the term "other algal components" include compounds other than fucoxanthin, fucoxanthinol, amarouciaxanthin A and isofucoxanthinol that may be eventually present in the algal extract of the invention, wherein said compounds are present in algae and are soluble in the solvent used for producing the algal extract of the invention. Said components may be naturally occurring compounds in algae, typically in fucoxanthin producing algae, such as metabolites, carotenes, chlorophylls, lipids, and the like, or not, for example, in case of engineered (e.g., transformed, etc.) algae that produce non-naturally occurring compounds in algae, or alternatively, said compounds may be produced during the process for producing the algal extract of the invention. In a particular embodiment, the solvent used for producing the algal extract of the invention is an alcohol, such as ethanol, and the algal extract of the invention can contain diadinoxanthin, diazoxanthin, β-carotene, cholorophylls, lipids, etc.

Illustrative, non-limitative, examples of lipids which can be present in the algal extract of the invention include fatty acids (i.e., carboxylic acids with a long aliphatic tail (chain), usually consisting of 4 to 28 carbon atoms, which is either saturated or unsaturated), including polyunsaturated fatty acids (PUFAs), i.e., fatty acids that contain more than one double bond in their backbone (although some monounsaturated omega-9 fatty acids are also considered as PUFAs). By illustrative, the algal extract of the invention can contain fatty acids such as, for example, caprylic acid (C8:0), capric acid (C10:0), undecanoic acid (C11:0 (IS)), lauric acid (C12:0), tridecanoic acid (C13:0), myristic acid (C14:0), myristoleic acid (C14:1 n5), pentadecanoic acid (C15:0), *cis*-10-pentadecenoic acid (C15:1 n5), palmitic acid (C16:0), palmitoleic acid (C16:1 n7), hexadecanoic acid (C17:0), *cis*-10-hexadecanoic acid (C17:1 n7), stearic acid (C18:0), elaidic acid (C18:1t n9), oleic acid (C18:1c n9), linolelaidic acid (C18:2t n6), linoleic acid [LA] (C18:2c n6), gamma-linoleic acid [GLA] (C18:3 n6), arachidic acid (C20:0), alpha-linolenic acid [ALA] (C18:3 n3), *cis*-11-eicosenoic acid (C20:1 n9), heneicosanoic acid (C21:0), *cis*-11,14-eicosadienoic acid (C20:2 n6), *cis*-8,11,14-eicosatrienoic acid (C20:3 n6), behenic acid (C22:0), arachidonic acid [ARA] (C20:4 n6), *cis*-11,14,17-eicosatrienoic acid (C20:3 n3), euricic acid (C22:1 n9), tricosanoic acid (C23:0), *cis*-5,8,11,14,17-eicosapentaenoic acid [EPA] (C20:5 n3), *cis*-13,16-docosadienoic acid (C22:2 n6), lignoceric acid (C24:0), nervonic acid (C:24:1 n9), cis-4,7,10,13,16-docosapentaenoic acid (C22:5 n6), *cis*-4,7,10,13,16,19-docosahexanoic acid [DHA] (C22:6 n3), etc.

The "other algal components" can be present in the algal extract of the invention in a very broad concentration range. Nevertheless, in a particular embodiment, fucoxanthinol is present in the algal extract of the invention at a concentration comprised between about 1% and about 99.998% by weight with respect to the total weight of the algal extract of the invention, preferably between 10% and 99.5% by weight, more preferably between 10% and 99% by weight, still more preferably between 30% and 98.5% by weight, even more preferably between 50% and 98% by weight. In some particular embodiments, the algal extract of the invention comprises 1% to 99.998% by weight of other algal components, for example, between 10% and 99.998% by weight, between 20% and 99.998% by weight, between 30% and 99.998% by weight, between 40% and 99.998% by weight, between 45% and 99.998% by weight, between 50% and 99.998% by weight, between 55% and 99.998% by weight, between 60% and 99.998% by weight, between 70% and 99.998% by weight, between 80% and 99.998% by weight, between 90% and 99.998% by weight. In some specific embodiments, the algal extract of the invention comprises between 80% and 98.5% by weight, between 89.7% and 96.9% by weight, or between 79.9% and 94.94% by weight, of the other algal components.

The amounts in which the different components that may be present in the other algal components fraction can vary broadly depending among other things on the algae, the solvent used for producing the extract, the extraction conditions, etc.

In a particular and preferred embodiment, the other algal component fraction that is present in the algal extract of the invention comprises one or more fatty acids, preferably one or more PUFAs, such as the above mentioned fatty acids and PUFAs, advantageously polyunsaturated omega-3 fatty acids such as ALA, *cis*-11,14,17-eicosatrienoic acid (C20:3 n3), EPA, DHA, etc., polyunsaturated omega-6 fatty acids such as LA, GLA, *cis*-11,14-eicosadienoic acid (C20:2 n6), *cis*-8,11,14-eicosatrienoic acid (C20:3 n6), ARA, *cis*-13,16-docosadienoic acid (C22:2 n6), cis-4,7,10,13,16-docosapentaenoic acid (C22:5 n6), etc., unsaturated omega-9 fatty acids such as palmitoleic acid (C16:1 n7), oleic acid (C18:1c n9), *cis*-11-eicosenoic acid (C20:1 n9), euricic acid (C22:1 n9), nervonic acid (C:24:1 n9), etc., and any combination thereof. The presence ofPUFAs, specially DHA, appears to increase the absorption and efficacy of fucoxanthin.

Thus in a particular embodiment, the algal extract of the invention comprises at least one fatty acid, either a saturated fatty acid or an unsaturated fatty acids. In another particular embodiment, the algal extract of the invention comprises at least a PUFA, for example, a polyunsaturated omega-3 fatty acid, a polyunsaturated omega-6 fatty acid, or an unsaturated omega-9 fatty acid. In another particular embodiment, the algal extract of the invention comprises two or more fatty acids, the fatty acids being saturated fatty acids or unsaturated fatty acids including PUFAs, in any combination thereof. In a preferred embodiment, the algal extract of the invention comprises a fatty acid selected from the group consisting of palmitoleic acid, oleic acid, LA, GLA, ALA, ARA, DHA and any combination thereof.

The fatty acids can be present in the algal extract of the invention (as a component of the "other algal components" fraction) in a very broad concentration range. Nevertheless, in a particular embodiment, fatty acids are present in the algal extract of the invention at a concentration comprised between about 1% and about 50% by weight with respect to the total weight of the algal extract of the invention, preferably between 2% and 40% by weight, more preferably between 3% and 30% by weight, still more preferably between 4% and 25% by weight, even more preferably between 5% and 20% by weight.

In a particular embodiment, the algal extract of the invention comprises:
- 0.001 % to 90% by weight of fucoxanthin;
- 0.001 % to 90% by weight of fucoxanthinol;
- 0% to 90% by weight of amarouciaxanthin A;
- 0% to 10% by weight of isofucoxanthinol; and
- 1% to 99.998% by weight of other algal components.

In a particular embodiment, the algal extract of the invention comprises:
- 0.001 % to 90% by weight of fucoxanthin;
- 0.001 % to 90% by weight of fucoxanthinol; and
- 1% to 99.998% by weight of other algal components.

In another particular embodiment, the algal extract of the invention comprises:
- 1% to 10% by weight of fucoxanthin;
- 0.1 % to 10% by weight of fucoxanthinol; and
- 80% to 98.5% by weight of other algal components.

In another particular embodiment, the algal extract of the invention comprises:
- 0.001 % to 90% by weight of fucoxanthin;
- 0.001 % to 90% by weight of fucoxanthinol;
- more than 0% to 90% by weight of amarouciaxanthin A; and
- 1% to 99.998% by weight of other algal components.

In another particular embodiment, the algal extract of the invention comprises:
- 0.001 % to 90% by weight of fucoxanthin;
- 0.001 % to 90% by weight of fucoxanthinol;
- more than 0% to 10% by weight of isofucoxanthinol; and
- 1% to 99.998% by weight of other algal components.

In another particular embodiment, the algal extract of the invention comprises:
- 0.001 % to 90% by weight of fucoxanthin;
- 0.001 % to 90% by weight of fucoxanthinol;
- more than 0% to 90% by weight of amarouciaxanthin A;
- more than 0% to 10% by weight of isofucoxanthinol; and
- 1% to 99.998% by weight of other algal components.

In another particular embodiment, the algal extract of the invention comprises:
- 0.001% to 15% by weight of fucoxanthin;
- 0.001% to 15% by weight of fucoxanthinol;
- 0% to 15% by weight of amarouciaxanthin A; and
- 55% to 99.998% by weight of other algal components.

In another particular embodiment, the algal extract of the invention comprises:
- 0.001% to 15% by weight of fucoxanthin;
- 0.001% to 15% by weight of fucoxanthinol;
- 0% to 15% by weight of amarouciaxanthin A; and
- 0% to 10% by weight of isofucoxanthinol;
- 45% to 99.998% by weight of other algal components.

In another particular embodiment, the algal extract of the invention comprises:
- 2% to 4% by weight of fucoxanthin;
- 0.4% to 0.8% by weight of fucoxanthinol;
- 0.6% to 4% by weight of amarouciaxanthin A;
- 0.1 % to 1.5 % by weight of isofucoxanthinol; and
- 89.7% to 96.9% by weight of other algal components.

In another particular embodiment, the algal extract of the invention comprises:
- 5% to 10% by weight of fucoxanthin;
- 0.02% to 0.1 % by weight of fucoxanthinol;
- 0.02% to 5% by weight of amarouciaxanthin A;
- 0.02% to 5% by weight of isofucoxanthinol; and
- 79.9% to 94.94% by weight of other algal components.

Any of the above particular embodiments of the algal extract of the invention, preferably comprises one or more fatty acids within the other algal components fraction, in an amount such that said fatty acids present in the algal extract of the invention are at a concentration comprised between about 1% and about 45% by weight with respect to the total weight of the algal extract of the invention, preferably between 2% and 40% by weight, more preferably between 3% and 30% by weight, still more preferably between 4% and 25% by weight, even more preferably between 5% and 20% by weight.

### Process for producing the algal extract of the invention

In another aspect, the invention relates to a process, hereinafter referred to as the "process of the invention", for producing an algal extract comprising fucoxanthin and fucoxanthinol, and, optionally, amarouciaxanthin A and/or isofucoxanthinol, together with other algal components (i.e., the algal extract of the invention), which comprises:
a) culturing fucoxanthin producing algae biomass under conditions that allow the production of fucoxanthin; and
b) contacting fucoxanthin with:
   b.1) a base under conditions that allow the hydrolysis of at least a portion of fucoxanthin to fucoxanthinol; or, alternatively, with
   b.2) an enzyme that catalyzes the conversion of fucoxanthin to fucoxanthinol under conditions that allow the conversion of at least a portion of fucoxanthin to fucoxanthinol.

An algal extract, as it has been previously mentioned, is a product obtained from algae.

As used herein, the term "alga" includes a large and diverse group of simple, typically autotrophic organisms, ranging from unicellular to multicellular forms, including both macroalgae and microalgae, i.e., microscopic algae, typically found in freshwater and marine systems.

According to the invention, the alga must produce fucoxanthin, either naturally or artificially (i.e., as a result of a process for transforming non-fucoxanthin producing algae into fucoxanthin-producing algae). Consequently, any fucoxanthin producing alga can be used in the process of the invention.

In a particular embodiment, the fucoxanthin producing alga is a macroalgae belonging to the phylum Rhodophyta or Pheophyta, or a microalgae belonging to the phylum Heterokontophyta, Haptophytas or Dinophyta [Takaichi S 2011. Carotenoids in Algae: Distributions, Blosyntehsis and Functions. Marine Drugs 9(6):1101-1118].

In a more particular embodiment, the fucoxanthin producing alga is a microalga belonging to a class selected from the group consisting of *Rapidophyceae, Bacillariophyceae* (Diatomeas), *Crysophyceae, Pavlophyceae,* and *Prymnesiophyceae.*

Still in a more particular embodiment, the fucoxanthin producing alga is a microalga that belongs to a genus selected from the group consisting of *Isochrysis, Thalassiosira, Navicula, Pavlova, Ochromonas, Phaeodactylum, Odontella, Skeletonema, Chaetoceros, Prymnesium, Nitzschia, Dinobryon, Synura, Chrysochromulina, Ochrosphaera, Cylindrotheca, Chromulina, Mallomonas,* and *Emiliania.*

Illustrative, non-limitative, examples of fucoxanthin producing algae include *Isochrysis aff. galbana, Thalassiosira pseudonana, Navicula incerta, Pavlova lutheri, Ochromonas* sp., *Phaeodactylum tricornutum, Odontella aurita, Isochrysis galbana, Isochrysis* sp., *Pavlova gyrans, Skeletonema costatus, Chaetoceros gracilis, Chaetoceros calcitrans, Prymnesium parvum, Nitzschia heufleriana, Nitzschia* sp., *Dinobryon* sp., *Synura uvella, Synura petersenii, Chrysochromulina brevifikum, Ochrosphaera neapolitana, Cylindrotheca fusiormis, Chromulina neblosa, Mallomonas asmundae, Ochromonas danica, Ochromonas spherocystis,* and *Emiliania huxleyi,* preferably, *I. aff. galbana, T. pseudonana, N. incerta, P. lutheri,* and *Ochromonas* sp.

The term "biomass", as used herein, includes biological material comprising, or deriving from, living or recently living organisms. By extension, the term includes not only the biological material or organic matter which constitutes an organism, but also the biological material or organic matter generated in a biological process, spontaneous or not spontaneous (i.e., provoked). Thus, the expression "fucoxanthin producing algae biomass" refers to biomass comprising fucoxanthin producing algae.

According to step a) of the process of the invention, the fucoxanthin producing algae biomass is cultured under conditions that allow the production of fucoxanthin. The conditions for culturing fucoxanthin producing algae can vary widely depending, among other factors, on the specific fucoxanthin producing alga elected for carrying out the process of the invention.

In a particular embodiment, the fucoxanthin producing alga is a photoautotroph organism (i.e., an organism capable of synthesizing its own food from inorganic substances using light as energy source and is capable of using carbon dioxide as its principal source of carbon). Thus, according to this particular embodiment, the fucoxanthin producing algae biomass can be obtained by photoautotrophic growth.

In another particular embodiment, the fucoxanthin producing alga is a mixotroph organism (i.e., an organism that can use a mix of different sources of energy and carbon). Thus, according to this particular embodiment, the fucoxanthin producing algae biomass can be obtained by mixotrophic growth.

For use within the context of the present invention, the fucoxanthin producing alga can be collected from the natural medium or can be cultured in a photobioreactor.

In a particular embodiment, the fucoxanthin producing alga is cultured in a photobioreactor in a suitable medium, under a suitable luminous intensity, at a suitable temperature. Practically any medium suitable for growing algae can be used; nevertheless, illustrative, non-limitative examples of said media include: f/2 (Guillard and ryther 1962. Studies of marine planktonic diatoms. I. Cyclotella nana Hustedt and Detonula confervacea Cleve. Can. J. Microbiol. 8:229-39), Erds (Tompkins et al., 1995. Culture Collection of Algae and Protozoa. Catalog of Strains. Ambleside, UK, 204 pp.) or K/2 (Keller et al., 1987 Media for the culture of oceanic ultraphytoplankton. J. Phycol. 23:633-8). The luminous intensity can vary widely, nevertheless, in a particular embodiment, the luminous intensity is comprised between 25 and 150 µmol fotons m⁻² s⁻¹. The temperature can vary usually between about 17°C and about 30°C. The culture can be performed in the absence of aeration or with aeration. In a particular embodiment, the culture is carried out without aeration. In another embodiment, the culture is performed with aeration, for example, with air or with up to 5% CO₂ enriched air, at a rate of delivery comprised between more than 0 and 1 L/min.

The fucoxanthin contained in the product resulting from step a) of the process of the invention is subjected to an alkaline chemical treatment or to an enzymatic hydrolysis [step b)].

Thus, in step b.1) of the process of the invention, fucoxanthin produced as in step a) is subjected to an alkaline treatment (saponification) under conditions that allow the hydrolysis of at least a portion of fucoxanthin to fucoxanthinol.

The base can be an inorganic base or an organic base. Illustrative, non-limitative, examples of inorganic bases include ammmonia (NH₃), alkaline metals or alkaline earth metals hydroxides or carbonates, such as, for example, KOH, Na₂CO₃, etc. Illustrative, non-limitative, examples of organic bases include N-heterocyclic compounds [e.g., benzimidazol, imidazole, piperidine, pyridine, etc., and derivatives thereof, such as 4-(dimethylaminopyridine), di-tert-butylpyridine, 2,6-lutidine, etc., 1,5-diazabicyclo [4.3.0]non-5-ene, 1,8-diazabicyclo[5.4.0]undec-7-ene, etc.], amines [e.g., methylamine, ethylamine, dimethylamine, diethylamine, diisopropylamine, N,N-diisopropylmethylamine, N-ethyldiisopropylamine, 2-(2chloro-6-fluorophenyl) ethylamine, choline, etc.], organolithiums [e.g., methyllithium, n-butyllithium, sec-butyllithium, tert-butyllithium, hexyllithium, etc.], Grignard reagents [e.g., butylmagnesium chloride, sec-butylmagnesium chloride, methylmagnesium bromide, ethylmagnesium bromide, 1,1-dimethylpropylmagnesium chloride, 2,2-dimethylpropylmagnesium chloride, methylmagnesium bromide, hexylmagnesium chloride, etc.], tetraalkylammonium hydroxides [e.g., tetramethylammonium hydroxide, tetramethylammonium hydroxide, diethyldimethylammonium hydroxide, benzyltrimethylammonium hydroxide, hexadecyltrimethylammonium hydroxide, etc.], phosphonium hydroxides [e.g., 2,8,9-trimethyl-2,5,8,9-tetraaza-1-phosphabicyclo [3.3.3]undecane, etc.], metal alkoxides [e.g., lithium methoxide, lithium ethoxide, lithium tert-butoxide, lithium isopropoxide, barium tert-butoxide, potassium tert-butoxide, magnesium di-tert-butoxide, etc.], metal amides [e.g., lithium diethylamide, lithium diisopropylamide, lithium bis(trimethylsilyl)amide, lithium dicyclohexylamide, lithium dimethylamide, etc.], metal silanoates [e.g., lithium trimethylsilanoate, etc.], phosphazene bases [e.g., phosphazene base P₁-t-Bu, etc.]. In a preferred embodiment, the inorganic base is KOH or NH₄OH, and the organic base is lithium tert-butoxide.

The base can be added to the fucoxanthin containing medium at any appropriate molar ratio with respect to fucoxanthin. In any case, the base will be added in a ratio with respect to fucoxanthin that allows the hydrolysis of at least a portion of fucoxanthin to fucoxanthinol. Usually the base is added at a ratio higher than the corresponding stoichiometric ratio because a portion of the base can react with other co-extracted algal components such as, for example, lipids or chlorophylls. Thus, the presence of said components should be taken into account when preparing the base to be added to the reaction medium.

The reaction between fucoxanthin and the base is carried out in the dark, in a suitable reaction medium comprising an organic solvent, at a suitable temperature, and for a suitable period of time (reaction time) so that at least a portion of fucoxanthin is converted to fucoxanthinol. Illustrative, non-limitative, examples of organic solvents include alcohols (e.g., methanol, ethanol, etc.), ethers (e.g., tetrahydrofuran (THF), etc.), ketones (e.g., acetone, etc.), etc. The temperature can vary usually between about 4°C and about 30°C. The period of time must be sufficient to guarantee that at least a portion of fucoxanthin is converted to fucoxanthinol; although it can vary within a broad range, in a particular embodiment, the reaction time between fucoxanthin and the base is comprised between about 2 minutes and 24 hours, preferably between 2 minutes and 12 hours, more preferably between 5 minutes and 6 hours, still more preferably between 10 minutes and 3 hours, usually between about 10 minutes and about 120 minutes. The reaction can be carried out under inert atmosphere or not. Thus, in a particular embodiment, the reaction is carried out under inert atmosphere. In another particular embodiment, the reaction is not carried out under inert atmosphere.

As it will be evident for the skilled person in the art, varying the hydrolysis conditions, it is possible to regulate or control the conversion of fucoxanthin to fucoxanthinol. In fact, varying the amount and type of base, reaction medium, reaction time and reaction temperature it is possible to control (e.g., increase) the conversion of fucoxanthin to fucoxanthinol. In all cases, the reaction is performed in the dark. Further, depending on the base and the reaction time, the reaction can be performed (or not) under inert atmosphere. By illustrative, when fucoxanthin is hydrolyzed using 4 g 1⁻¹ of KOH in ethanol, at 25°C during 10 minutes under a non-inert atmosphere, the final algal extract may contain 3% by weight of fucoxanthin, 0.3% by weight of fucoxanthinol, 1.3% by weight of amarouciaxanthin A and 1% by weight of isofucoxanthinol. On the other hand, when fucoxanthin is hydrolyzed using 6 g 1⁻¹ of KOH in methanol, at 0°C during 60 minutes under inert atmosphere, the final algal extract may contain 5% by weight of fucoxanthin, 1.5% by weight of fucoxanthinol, 2% by weight of amarouciaxanthin A and 1% by weight of isofucoxanthinol.

In step b.2) of the process of the invention, fucoxanthin produced as in step a) is subjected to enzymatic hydrolysis with an enzyme that catalyzes the conversion of fucoxanthin to fucoxanthinol under conditions that allow the conversion of at least a portion of fucoxanthin to fucoxanthinol.

Enzymes that catalyze the conversion of fucoxanthin to fucoxanthinol include hydrolases (i.e., enzymes that act on ester bonds) included within class EC 3.1 class. Illustrative, non-limitative, examples of enzymes suitable or use in the process of the invention include lipases and cholesterol esterase. A lipase is an enzyme that catalyzes the hydrolysis of fats (lipids) and is considered as a subclass of esterases. A cholesterol esterase is an enzyme that catalyzes the hydrolysis of sterol esters into their component sterols and fatty acids. Preferred enzymes for use in the process of the invention include a pancreas lipase, such as a porcine pancreas lipase, or a cholesterol esterase.

Once finished the alkaline chemical treatment or enzymatic hydrolysis performed in step b), an algal extract comprising fucoxanthin and fucoxanthinol, is obtained. If desired, the algal extract comprising fucoxanthin and fucoxanthinol obtained is separated (removed) by conventional techniques including, for example, decantation after formation of a biphasic system, and, if desired, dried by rotoevaporation to dryness in order to obtain a substantially dry algal extract comprising fucoxanthin and fucoxanthinol, such as an algal extract of the invention, or concentrated by rotoevaporation and addition of a matrix oil, for example a vegetal (vegetable) oil (e.g., corn oil, etc.).

Thus, in a particular embodiment, the product resulting from the alkaline chemical treatment or enzymatic hydrolysis performed in step b) is treated with an aqueous saline solution in order to form a biphasic system comprising an aqueous phase and an organic phase, wherein the organic phase comprises fucoxanthin and fucoxanthinol.Virtually any non-toxic, water-soluble salt, can be used, preferably a non-toxic, water-soluble salt, that increases polarity of the aqueous phase, e.g., an aqueous solution comprising NaCl, KCl, NaHPO₃, etc. The organic phase is separated from the aqueous phase by any suitable technique, for example, decantation, centrifugation, etc., and, if desired, dried by rotoevaporation to dryness, in order to obtain a substantially dry algal extract comprising fucoxanthin and fucoxanthinol, such as an algal extract of the invention.

In a particular embodiment, the algal extract of the invention obtained according to the process of the invention comprises fucoxanthin and fucoxanthinol, and, optionally, amarouciaxanthin A and/or isofucoxanthinol, together with other algal components.

In another particular embodiment, the algal extract of the invention obtained according to the process of the invention comprises:
- 0.001 % to 90% by weight of fucoxanthin;
- 0.001 % to 90% by weight of fucoxanthinol;
- 0% to 90% by weight of amarouciaxanthin A;
- 0% to 10% by weight of isofucoxanthinol; and
- 1% to 99.998% by weight of other algal components.

In a particular embodiment, the algal extract of the invention so obtained comprises one or more fatty acids within the other algal components fraction, in an amount such that said fatty acids present in the algal extract of the invention are at a concentration comprised between about 1% and about 45% by weight with respect to the total weight of the algal extract of the invention, preferably between 2% and 40% by weight, more preferably between 3% and 30% by weight, still more preferably between 4% and 25% by weight, even more preferably between 5% and 20% by weight.

Other algal extracts included within the scope of the algal extract of invention obtainable according to the process of the invention include the algal extracts previously disclosed in connection with the algal extract of the invention and are included herein by reference.

The composition of the algal extract of the invention depends on several factors, for example, on the algae used as starting material, the solvent used in the extraction step, as well as on the treatment (chemical or enzymatic) applied on the product resulting from step a) of the process of the invention. Thus, if said product is subjected to the alkaline chemical treatment the algal extract of the invention may contain in addition to fucoxanthin and fucoxanthinol, amaurociaxanthin A and/or isofucoxanthinol, together with other algal compounds such as diadinoxanthin, diazoxanthin, β-carotene, chlorophylls, lipids, such as fatty acids and PUFAs, depending on the solvent used in the production of the extract. However, if the product resulting from step a) is subjected to the enzymatic hydrolysis the algal extract of the invention does not contain amaurociaxanthin A or isofucoxanthinol, but it contains fucoxanthin and fucoxanthinol, together with, optionally, other algal components such as diadinoxanthin, diazoxanthin, β-carotene, chlorophylls, lipids, such as fatty acids and PUFAs, depending on the solvent used in the production of the extract

The invention also contemplates the possibility of subjecting the product resulting from step a) of the process of the invention to a suitable preparation treatment in order to prepare fucoxanthin for the alkaline chemical treatment or enzymatic hydrolysis to be performed in step b) prior to perform step b) of the process of the invention.

Thus, in a particular embodiment, the algal biomass that contains fucoxanthin obtained by culturing fucoxanthin producing algae biomass under conditions that allow the production of fucoxanthin [i.e., the product resulting from step a) of the process of the invention] is removed from the culture medium, optionally dried, and subjected to a treatment, hereinafter referred to as "preparation treatment" comprising:
i) contacting said removed algal biomass containing fucoxanthin with an organic solvent in order to obtain an organic extract comprising fucoxanthin; and, if desired,
ii) drying said organic extract comprising fucoxanthin from step i) to obtain a residue comprising fucoxanthin; and
iii) dispersing (suspending) said residue comprising fucoxanthin from step ii) in an organic solvent.

The algal biomass that contains fucoxanthin obtained by culturing fucoxanthin producing algae biomass under conditions that allow the production of fucoxanthin can be removed by conventional means and techniques including, for example, filtration, centrifugation, flocculation or decantation. Further, if desired, the removed algal biomass containing fucoxanthin can be dried by freeze-drying, also known as lyophilisation or cryodesiccation or dried during 24 hours under vacuum at 50°C. The optionally dried algal biomass containing fucoxanthin is then contacted with an organic solvent in order to obtain an organic extract comprising fucoxanthin [step i)]. Practically any organic solvent in which fucoxanthin is at least partially soluble can be used; illustrative, non-limitative, examples of said organic solvents include alcohols (e.g., methanol, ethanol, etc.), ethers (e.g., diethyl ether, THF, etc.), etc. The organic extract comprising fucoxanthin is then dried to obtain a residue comprising fucoxanthin [step ii)] by rotoevaporation, and the resulting residue is dispersed in an organic solvent wherein fucoxanthin is at least partially soluble such as, for example, an alcohol (e.g., methanol, ethanol, etc.), an ether (e.g., diethyl ether, THF, etc.), etc., thus rendering an organic suspension comprising fucoxanthin. The resulting product can be subjected to the alkaline treatment or enzymatic hydrolysis as defined in step b) of the process of the invention.

In another particular embodiment, the algal biomass that contains fucoxanthin obtained by culturing fucoxanthin producing algae biomass under conditions that allow the production of fucoxanthin [i.e., the product resulting from step a) of the process of the invention] is not removed from the culture medium. This option can be followed when the treatment selected comprises the alkaline chemical treatment of fucoxanthin. Thus, according to this embodiment, the appropriate reagents and solvents for performing said treatment are added to the culture medium.

In another particular embodiment, the algal biomass that contains fucoxanthin obtained by culturing fucoxanthin producing algae biomass under conditions that allow the production of fucoxanthin [i.e., the product resulting from step a) of the process of the invention] is removed from the culture medium. This option is particularly useful when the treatment selected comprises the enzymatic hydrolysis of fucoxanthin (wherein the enzymatic hydrolysis is carried out in the resulting extract) or when the treatment selected comprises the alkaline chemical treatment of fucoxanthin. Thus, according to this embodiment, the appropriate enzymes, or alternatively alkaline reagents, for performing the corresponding treatments are added to the culture medium.

In a particular embodiment of the process of the invention, the invention contemplates a process for producing an algal extract comprising fucoxanthin and fucoxanthinol, and, optionally, amarouciaxanthin A and/or isofucoxanthinol, together with other algal components (i.e., the algal extract of the invention), hereinafter referred to as the "process [1] of the invention", which comprises:
a) culturing fucoxanthin producing algae biomass under conditions that allow the production of fucoxanthin;
b) removing the algal biomass comprising fucoxanthin resulting from step a);
c) contacting the algal biomass containing fucoxanthin resulting from step b) with an organic solvent under conditions that allow obtaining an organic extract comprising fucoxanthin; and, if desired, drying said organic extract comprising fucoxanthin to obtain a residue comprising fucoxanthin; and dispersing said residue comprising fucoxanthin in an organic solvent;
d) contacting the product resulting from step c) with fucoxanthin with:
   d.1) a base under conditions that allow the hydrolysis of at least a portion of fucoxanthin to fucoxanthinol; o, alternatively, with
   d.2) an enzyme that catalyzes the conversion of fucoxanthin to fucoxanthinol under conditions that allow the conversion of at least a portion of fucoxanthin to fucoxanthinol;
e) adding an aqueous saline solution to the product resulting from step d) under conditions that allow the formation of an aqueous phase and an organic phase, wherein the organic phase comprises fucoxanthin and fucoxanthinol;
f) removing and, if desired, drying said organic phase comprising fucoxanthin and fucoxanthinol, to render an extract comprising fucoxanthin and fucoxanthinol.

The particulars of the fucoxanthin producing algae biomass to be used in the process [1] of the invention have been previously described in connection with the process of the invention and are incorporated herein by reference.

According to step b) of the process [1] of the invention, the algal biomass comprising fucoxanthin resulting from step a) is removed by conventional techniques including, for example, filtration, floculation or centrifugation; if desired, the resulting product can be dried by, for example, freeze-drying or during 24 hours under vacuum at 50°C.

Step c) of the process [1] of the invention corresponds to the "preparation treatment" whose particulars have been previously described in connection with the process of the invention and are incorporated herein by reference.

Step d) of the process [1] of the invention corresponds to step b) of the process of the invention whose particulars have been previously described in connection with the process of the invention and are incorporated herein by reference.

According to step e) of the process [1] of the invention, an aqueous saline solution is added to the product resulting from step d) under conditions that allow the formation of an aqueous phase and an organic phase, wherein the organic phase comprises fucoxanthin and fucoxanthinol. Virtually any non-toxic, water-soluble salt, can be used, preferably a non-toxic, water-soluble salt, that increases polarity of the aqueous phase, e.g., an aqueous solution comprising NaCl, KCl, NaHPO₃, etc.

The product resulting from step e) of the process [1] of the invention, the organic phase comprising fucoxanthin and fucoxanthinol is separated from the aqueous phase by conventional techniques including, for example, centrifugation, decantation, and the organic phase is dried by rotoevaporation to dryness in order to render an algal extract comprising fucoxanthin and fucoxanthinol, such as an algal extract of the invention.

In another particular embodiment of the process of the invention, the invention contemplates a process for producing an algal extract comprising at least 37% by weight of fucoxanthin and 25% by weight of fucoxanthinol, and, optionally, amarouciaxanthin A and/or isofucoxanthinol, together with other algal components (i.e., an algal extract of the invention), hereinafter referred to as the "process [2] of the invention", which comprises:
a) culturing a fucoxanthin producing microalgal under conditions that allow the production of fucoxanthin;
b) removing, and optionally drying, said microalgal biomass comprising fucoxanthin resulting from step a);
c) contacting said microalgal biomass comprising fucoxanthin from step b) with ethanol under conditions that allow obtaining an ethanol extract comprising fucoxanthin;
d) drying said ethanol extract comprising fucoxanthin from step c) to obtain a solid residue comprising fucoxanthin;
e) dispersing said solid residue comprising fucoxanthin from step d) in diethyl ether;
f) contacting the suspension resulting from step e) with a base in a medium comprising an alcohol;
g) adding an aqueous sodium chloride solution to the product resulting from step f) under conditions that allow the formation of an aqueous phase and an organic phase, wherein the organic phase comprises fucoxanthin and fucoxanthinol;
h) removing said organic phase comprising fucoxanthin and fucoxanthinol from step g);
i) washing said removed organic phase comprising fucoxanthin and fucoxanthinole; and
j) drying said organic phase comprising fucoxanthin and fucoxanthinol to render an extract comprising at least 37% by weight of fucoxanthin and 25% by weight of fucoxanthinol.

The particulars of the fucoxanthin producing microalgae biomass to be used in the process [2] of the invention have been previously described in connection with the process of the invention and are incorporated herein by reference. In a particular embodiment, said fucoxanthin producing microalga is a microalga belonging to the phylum Heterokontophyta, Haptophytas or Dinophyta, such as a microalga selected from the group consisting of *Isochrysis aff. galbana, Thalassiosira pseudonana, Navicula incerta, Pavlova lutheri, Ochromonas* sp., *Phaeodactylum tricornutum, Odontella aurita, Isochrysis galbana, Isochrysis* sp., *Pavlova gyrans, Skeletonema costatus, Chaetoceros gracilis, Chaetoceros calcitrans, Prymnesium parvum, Nitzschia heufleriana, Nitzschia* sp., *Dinobryon* sp., *Synura uvella, Synura petersenii, Chrysochromulina brevifikum, Ochrosphaera neapolitana, Cylindrotheca fusiformis, Chromulina neblosa, Mallomonas asmundae, Ochromonas danica, Ochromonas spherocystis,* and *Emiliania huxleyi,* preferably, *I. aff. galbana, T. pseudonana, N. incerta, P. lutheri,* and *Ochromonas* sp.

In a particular embodiment, the fucoxanthin producing microalga is cultured in a photobioreactor in a suitable medium, such as f/2, under a luminous intensity comprised between 25 and 150 µmol fotons m⁻² s⁻¹, at a temperature between about 17°C and about 30°C, optionally with aeration. In a particular embodiment, the culture is carried out with aeration, for example, with air or with up to 5% CO₂ enriched air, at a rate of delivery comprised between more than 0 and 1 L/min.

According to step b) of the process [2] of the invention, the microalgal biomass comprising fucoxanthin resulting from step a) is removed by conventional techniques including, for example, filtration, floculation or centrifugation, and, optionally, dried by freeze-drying or during 24 hours under vacuum at 50°C.

The dry microalgal biomass resulting from step c) is contacted with ethanol in step c) of the process [2] of the invention under conditions that allow obtaining an ethanol extract comprising fucoxanthin. Said ethanol extract comprising fucoxanthin is dried [step d) of the process [2] of the invention] by rotoevaporation to dryness in order to obtain a residue comprising fucoxanthin. The residue comprising fucoxanthin is dispersed [step e) of the process [2] of the invention] in an ether, such as diethyl ether, in order to obtain an diethyl ether extract comprising fucoxanthin.

The diethyl ether extract comprising fucoxanthin resulting from step d) is subjected to an alkaline hydrolysis [step f)] by treatment con a base, such as an alkaline hydroxide, preferably NaOH or KOH, in a medium comprising an organic solvent, such as an alcohol, preferably methanol, in inert atmosphere, at dark, at a temperature comprised between about 0° and about 60°C, in order to provoke the conversion of at least a portion of fucoxanthin to fucoxanthinol. 1.

According to step g) of the process [2] of the invention, an aqueous salt solution comprising sodium chloride is added to the product resulting from step f) comprising fucoxanthin and fucoxanthinol under conditions that allow the formation of an aqueous phase and an organic phase, wherein the organic phase comprises fucoxanthin and fucoxanthinol. The organic phase comprising fucoxanthin and fucoxanthinol is separated [step h)] from the aqueous phase by conventional means, for example by decantation, and the organic phase is washed with an aqueous sodium chloride solution [step i)] and dried [step j)] by rotoevaporation to dryness to render an algal extract comprising at least 37% by weight of fucoxanthin and 25% by weight of fucoxanthinol.

### Applications of the algal extract of the invention

The algal extract comprising fucoxanthin and fucoxanthinol, and, optionally, amarouciaxanthin A and/or isofucoxanthinol, together with other algal components, can be used in a lot of applications. Some applications relate to its use as anti-inflammatory agent whereas some other applications relate to the uses of the components thereof; by illustrative, it is well-known that fucoxanthin and fucoxanthinol can be used as anti-obesity agents since they have a fat burner effect, antiangiogenic agents, anti-neoplastic agents, neovascularization inhibitors, adiponectin production accelerators, cholesterol lowering agents, antihypertensive agents, antiallergic agents, etc., as well as in the treatment of skin pigmentation, metabolic syndrome management, or in the treatment and/or prevention of virus associated malignancy such as adult T-cell leukemia and Burkitt lymphoma, hyperuricemia, osteoporosis, depression, etc. Thus, said algal extract can be used in a lot of cosmetic, cosmeceutical, food, nutraceutical, or pharmaceutical applications.

Thus, in a particular embodiment, the algal extract of the invention can be used as an adiponectin production accelerator, an antiangiogenic agent, an antiallergic agent, an antiangiogenic agent, an antibacterial agent, an anticancer agent, an antidiabetic agent, an antifungal agent, an antiinflamatory agent, an antimalarial agent, an antineoplastic agent, an antiobesity agent, an antioxidant agent, an antihypertensive agent, a bone protective agent, a cerebrovascular protective agent, a cholesterol lowering agent, an hepatoprotective agent, an ocular protective agent, a neovascularization inhibitor, a skin-protective agent (specially to protect skin against UV-B radiation photodamage and UV-B radiation induced skin pigmentation) as well as in the prevention and/or treatment of depression, hyperuricemia, inflammatory diseases (e.g., gastrointestinal inflammatory diseases (Crohn's Disease, etc.), gingivitis, joint/muscle recovery from overexertion, osteoarthritis, psoriasis, rheumatoid arthritis, etc.), metabolic syndrome management, osteoporosis, skin pigmentation, virus associated malignancy such as adult T-cell leukemia and Burkitt lymphoma, etc.

Thus, the algal extract of the invention can be used in a lot of cosmetic, cosmeceutical, food, nutraceutical, or pharmaceutical applications.

Therefore in another aspect, the invention relates to a composition, hereinafter "composition of the invention", comprising an algal extract of the invention, and a cosmetic, cosmeceutical, food, nutraceutical, or pharmaceutical acceptable vehicle.

The particulars of the algal extract of the invention have already been defined above and are incorporated herein by reference.

In a particular embodiment, the composition of the invention is a cosmetic or personal care composition comprising an algal extract of the invention together with a cosmetically acceptable vehicle. As used herein, the term "cosmetic composition" or "personal care composition" refers to a composition suitable for use in personal hygiene of human beings or animals, or in order to enhance the natural beauty or change the body appearance without affecting the structure or functions of the human or animal body, comprising one or more products providing such effects. If desired, the cosmetic composition provided by the invention can contain, in addition to the algal extract of the invention, one or more cosmetic products, i.e., substances or mixtures intended to be placed in contact with the external parts of the human or animal body (epidermis, hair system, nails, lips and external genital organs) or with the teeth and the buccal mucosa, for the exclusive or main purpose of cleaning them, perfuming them, changing their appearance, protecting them, keeping them in good condition or correcting body odors. Illustrative examples of cosmetic products include the products contained in the INCI (International Nomenclature of Cosmetic Ingredients) list. Cosmetic or personal care compositions include products such as balms, pads, pomades, creams, etc.

In a particular embodiment, the cosmetic or personal care composition provided by the present invention comprises an algal extract of the invention and an acceptable oral or topical carrier therefor. In a more particular embodiment, the alga is a microalga, such as any of the microalgae mentioned in connection with the algal extract of the invention such as a microalga belonging to a class selected from the group consisting of *Rapidophyceae, Bacillariophyceae* (Diatomeas), *Crysophyceae, Pavlophyceae,* and *Prymnesiophyceae,* specially, a microalga that belongs to a genus selected from the group consisting of *Isochrysis, Thalassiosira, Navicula, Pavlova, Ochromonas, Phaeodactylum, Odontella, Skeletonema, Chaetoceros, Prymnesium, Nitzschia, Dinobryon, Synura, Chrysochromulina, Ochrosphaera, Cylindrotheca, Chromulina, Mallomonas,* and *Emiliania.*

In another particular embodiment, the cosmetic or personal care composition provided by the present invention comprises between 0.1% and 5% by weight of an algal extract of the invention, for example, of an algal extract of the invention wherein the alga is a microalga.

In another particular embodiment, the cosmetic or personal care composition provided by the present invention comprises a sunscreen agent. Illustrative, non-limitative, examples of sunscreens include microfine titanium dioxide; microfine zinc oxide; boron nitride; p-aminobenzoic acids, esters and derivatives thereof; methoxycinnamate esters; benzophenones; 2-phenylbenzimidazole-5- sulfonic acid; disodium phenyl dibenzimidazole tetrasulfonate; terphthalylidene dicamphor sulfonic acid; alkyl- , - diphenylacrylates; triazine sunscreens; benztriazolyl sunscreens; camphor sunscreens; organic pigment sunscreens; silicone sunscreens; and salicylate sunscreens.

In another particular embodiment, the algal extract of the invention in the cosmetic or personal care composition provided by the present invention is contained in an aqueous phase of said composition.

In another particular embodiment, the cosmetic or personal care composition provided by the present invention comprises an oil-in-water emulsion.

In another particular embodiment, the cosmetic or personal care composition provided by the present invention for application to the skin in the form of a cream emulsion, gel, milk, suspension, O/W emulsion, W/O emulsion, liposome foam, aqueous or emulsion lotion, spray or a waxy stick.

In another particular embodiment, the cosmetic or personal care composition provided by the present invention can be used in the prevention, amelioration or treatment of damage of mammalian skin. In another particular embodiment, the cosmetic or personal care composition provided by the present invention can be used for inhibiting one or more of the following skin conditions: inflammation, photo-ageing skin damage, uneven pigmentation, wrinkles and sagging skin.

In another particular embodiment, the composition of the invention is a cosmeceutical composition comprising an algal extract of the invention together with a cosmeceutically acceptable vehicle. As used herein, the term "cosmeceutical composition" refers to a composition suitable for use in the body or animal body comprising one or more cosmeceutical products (functional cosmetics, dermoceuticals or active cosmetics), i.e., topical hybrid products with cosmetical-pharmaceutical characteristics containing active ingredients having effect on user's skin, hair and/or nails, at higher and more effective concentrations, therefore they are located in an intermediate level between cosmetic and drug. Illustrative examples of cosmeceutical products include essential oils, ceramides, enzymes, minerals, peptides, vitamins, etc. The person skilled in the art will understand that the algal extract of the invention or the compositions containing them can be part of a food or feed, or of a nutraceutical, pharmaceutical, or cosmeceutical product, which constitutes an additional aspect of the present invention. Said products can be in a liquid, semi-solid or solid form.

In another particular embodiment, the composition of the invention is a food or feed comprising an algal extract of the invention. As used herein, the term "food" is any substance or product of any nature, solid or liquid, natural or processed which due to its characteristics, applications, components, preparation and state of preservation, can usually or ideally be used for some of the following purposes: a) as normal nutrition for human beings or animals or as pleasurable foods; or b) as dietetic products, in special cases of human or animal food. The term "feed" includes all the natural materials and finished products of any origin which, separately or conveniently mixed with one another, are suitable as animal food. A ready-to-eat food is that which does not need to be diluted by means of an aqueous solution suitable for consumption for example. In principle, the ingredients present in a ready-to-eat food are balanced and there is no need to add additional ingredients to the food to make it ready to eat, such considered by a person skilled in the art. A concentrated food is that in which one or more ingredients are present at a higher concentration than in a ready-to-eat food, therefore for use it is necessary to dilute it by means of an aqueous solution suitable for consumption for example. Non-limiting, illustrative examples of foods provided by this invention include both dairy products and derivatives, for example, fermented milks, yoghurt, kephir, curd, cheeses, butters, ice creams, milk-based desserts, etc., and non-dairy products, such as baked products, cakes and pastries, cereals, chocolates, jams, juices, other fruit derivatives, oils and margarines, prepared dishes, etc. In particular embodiment, the food comprises between 0.1 % and 5% by weight of an algal extract of the invention.

In another particular embodiment, the composition of the invention is a nutraceutical composition comprising an algal extract of the invention together with a nutraceutically acceptable vehicle. As used herein, the term "nutraceutical composition" refers to a composition suitable for use in human beings or animals, comprising one or more natural products with therapeutic action which provide a health benefit or have been associated with disease prevention or reduction, for example, fucoxanthin, fucoxanthinol, etc., and it includes dietary supplements presented in a non-food matrix (e.g., capsules, powder, etc.) of a concentrated natural bioactive product usually present (or not) in the foods and which, when taken in a dose higher than that existing in those foods, exerts a favorable effect on health which is greater than effect which the normal food may have. Therefore, the term "nutraceutical composition" includes isolated or purified food products as well as additives or food supplements which are generally presented in dosage forms normally used orally, for example, capsules, tablets, sachets, drinkable phials, etc.; such products provide a physiological benefit or protection against diseases, generally against chronic diseases. If desired, the nutraceutical composition provided by the invention can contain, in addition to the algal extract of the invention, one or more nutraceuticals (products or substances associated with disease prevention or reduction), for example, flavonoids, omega-3 fatty acids, etc., and/or one or more prebiotics (non-digestible food ingredients which stimulate probiotic activity and/or growth), for example, oligofructose, pectin, inulin, galacto-oligosaccharides, lactulose, human milk oligosaccharides, dietary fiber, etc.

In a particular embodiment, the nutraceutical composition provided by the present invention comprises an algal extract of the invention and an acceptable oral carrier therefor. In a more particular embodiment, the alga is a microalga, such as any of the microalgae mentioned in connection with the algal extract of the invention. In another particular embodiment, the nutraceutical composition provided by the present invention comprises between 0.1% and 5% by weight of an algal extract of the invention, for example, of an algal extract of the invention wherein the alga is a microalga. In another particular embodiment, the algal extract of the invention in the nutraceutical composition provided by the present invention is contained in an aqueous phase of said composition. In another particular embodiment, the nutraceutical composition provided by the present invention comprises an oil-in-water emulsion.

In another particular embodiment, the composition of the invention is a pharmaceutical composition comprising an algal extract of the invention together with a pharmaceutically acceptable vehicle, suitable for oral, topical, rectal or vaginal administration; to that end, said composition comprises a pharmaceutically acceptable vehicle comprising one or more excipients suitable for oral administration, for example, in the form of capsule, powder, granulate, tablet (coated or non-coated), sachet, matrix, suspension, etc., or a pharmaceutically acceptable vehicle comprising one or more excipients suitable for topical administration, for example, in the form of cream, ointment, salve, etc., or a pharmaceutically acceptable vehicle comprising one or more excipients suitable for rectal administration, for example, in the form of suppository, etc., or a pharmaceutically acceptable vehicle comprising one or more excipients suitable for vaginal administration, for example, in the form of bolus, suppository, etc. Information about excipients suitable for the formulation of pharmaceutical compositions intended for oral, topical, rectal or vaginal administration, as well as about the production of said pharmaceutical compositions can be found in the book "Tratado de Farmacia Galénica", by C. Faulí i Trillo, 10th Edition, 1993, Luzán 5, S.A. de Ediciones.

As used herein, the term "subject" includes any mammal animal including human being.

In a particular embodiment, the pharmaceutical composition provided by the present invention comprises an algal extract of the invention and an acceptable oral carrier therefor. In a more particular embodiment, the alga is a microalga, such as any of the microalgae mentioned in connection with the algal extract of the invention. In another particular embodiment, the pharmaceutical composition provided by the present invention comprises between 0.1% and 5% by weight of an algal extract of the invention, for example, of an algal extract of the invention wherein the alga is a microalga. In another particular embodiment, the algal extract of the invention in the pharmaceutical composition provided by the present invention is contained in an aqueous phase of said composition. In another particular embodiment, the pharmaceutical composition provided by the present invention comprises an oil-in-water emulsion.

In another particular embodiment, the pharmaceutical composition provided by the present invention comprises a sunscreen agent such as any of the sunscreen agents mentioned on connection with the cosmetic composition provided by this invention.

In another particular embodiment, the algal extract of the invention in the pharmaceutical composition provided by the present invention is contained in an aqueous phase of said composition. In another particular embodiment, the pharmaceutical composition provided by the present invention comprises an oil-in-water emulsion. In another particular embodiment, the pharmaceutical composition provided by the present invention for application to the skin in the form of a cream emulsion, gel, milk, suspension, O/W emulsion, W/O emulsion, liposome foam, aqueous or emulsion lotion, spray or a waxy stick.

In another particular embodiment, the pharmaceutical composition provided by the present invention can be used in the prevention, amelioration or treatment of damage of mammalian skin. In another particular embodiment, the pharmaceutical composition provided by the present invention can be used for inhibiting one or more of the following skin conditions: inflammation, photo-ageing skin damage, uneven pigmentation, wrinkles and sagging skin.

In another aspect, the invention relates to an algal extract of the invention for use as an adiponectin production accelerator, an antiangiogenic agent, an antiallergic agent, an antiangiogenic agent, an antibacterial agent, an anticancer agent, an antidiabetic agent, an antifungal agent, an antiinflamatory agent, an antimalarial agent, an antineoplastic agent, an antiobesity agent, an antioxidant agent, an antihypertensive agent, a bone protective agent, a cerebrovascular protective agent, a cholesterol lowering agent, an hepatoprotective agent, an ocular protective agent, a neovascularization inhibitor, a skin-protective agent (specially to protect skin against UV-B radiation photodamage and UV-B radiation induced skin pigmentation); or, expressed in an alternative way, the invention also relates to the use of an algal extract of the invention as an adiponectin production accelerator, an antiangiogenic agent, an antiallergic agent, an antiangiogenic agent, an antibacterial agent, an anticancer agent, an antidiabetic agent, an antifungal agent, an antiinflamatory agent, an antimalarial agent, an antineoplastic agent, an antiobesity agent, an antioxidant agent, an antihypertensive agent, a bone protective agent, a cerebrovascular protective agent, a cholesterol lowering agent, an hepatoprotective agent, an ocular protective agent, a neovascularization inhibitor, a skin-protective agent (specially to protect skin against UV-B radiation photodamage and UV-B radiation induced skin pigmentation).

In another aspect, the invention relates to an algal extract of the invention for use in the prevention and/or treatment of cancer, depression, hyperuricemia, inflammatory diseases (e.g., gastrointestinal inflammatory diseases (Crohn's Disease, etc.), gingivitis, joint/muscle recovery from overexertion, osteoarthritis, psoriasis, rheumatoid arthritis, etc.), metabolic syndrome management, osteoporosis, skin pigmentation, virus associated malignancy such as adult T-cell leukemia and Burkitt lymphoma; or for the prevention, amelioration or treatment of damage of mammalian skin, particularly, for inhibiting one or more of the following skin conditions: inflammation, photo-ageing skin damage, uneven pigmentation, wrinkles and sagging skin.

Alternatively, the invention also relates to the use of an algal extract of the invention in the manufacture of a pharmaceutical composition for the prevention and/or treatment of cancer, depression, hyperuricemia, inflammatory diseases (e.g., gastrointestinal inflammatory diseases (Crohn's Disease, etc.), gingivitis, joint/muscle recovery from overexertion, osteoarthritis, psoriasis, rheumatoid arthritis, etc.), metabolic syndrome management, osteoporosis, skin pigmentation, virus associated malignancy such as adult T-cell leukemia and Burkitt lymphoma, or for the prevention, amelioration or treatment of damage of mammalian skin, particularly, for inhibiting one or more of the following skin conditions: inflammation, photo-ageing skin damage, uneven pigmentation, wrinkles and sagging skin.

The invention also relates to the treatment of a disease which comprises administering to a subject in need thereof a therapeutically effective amount of an algal extract of the invention, wherein said disease is cancer, depression, hyperuricemia, inflammatory diseases (e.g., gastrointestinal inflammatory diseases (Crohn's Disease, etc.), gingivitis, joint/muscle recovery from overexertion, osteoarthritis, psoriasis, rheumatoid arthritis, etc.), metabolic syndrome management, osteoporosis, skin pigmentation, virus associated malignancy such as adult T-cell leukemia and Burkitt lymphoma.

The invention also relates to the treatment of damage of mammalian skin, particularly, for inhibiting one or more of the following skin conditions: inflammation, photo-ageing skin damage, uneven pigmentation, wrinkles and sagging skin, which comprises administering to a subject an effective amount of an algal extract of the invention.

The particulars of the algal extract of the invention have already been defined above and are incorporated herein by reference.

The following examples illustrate the invention and must not be considered as limiting same.

### EXAMPLE 1

### Purified fucoxanthin chemical and enzymatic derivatization (derivatization process controls)

In the chemical derivatization process, 5 mg of pure fucoxanthin (purity > 95%, Sigma-Aldrich) were dissolved in diethyl ether. One volume of methanol containing 10 g 1⁻¹ of potassium hydroxide was added. Saponification reaction was carried out under nitrogen atmosphere, in the dark, and at 0°C, during 60 minutes. To stop the reaction, a volume, equivalent to that of the diethyl ether extract, of water containing 100 g l⁻¹ of sodium chloride was added to the mixture. Two phases were obtained. The upper phase (containing the carotenoids dissolved in diethyl ether) was recovered by decantation. This organic extract was neutralized twice by addition of two more volumes of water containing 100 g l⁻¹ of sodium chloride and agitation, in order to remove traces of methanol and potassium hydroxide. Finally, diethyl ether was eliminated by rotoevaporation. This chemical derivatization process, when applied to algae extracts, was named "C-MEDPA Derivatization Process", wherein MEDPA means "(Micro)algae Extracts Derivatization Platform to Actives".

In the enzymatic derivatization approach, 5 mg of purified fucoxanthin were suspended with the same weight of taurocholic acid in aqueous phosphate buffer (PBS) pH 7 containing 50 mg of pig liver lipase per gram of pigment (100-400 units/g, Sigma-Aldrich). Alternatively, 10 units of cholesterol esterase from *Pseudomonas fluorescens* (Sigma-Aldrich) per gram of biomass were used. The extract was incubated at 37°C for 24 hours. Carotenoids were extracted with subsequent 1:1 volumes of diethyl ether, repeating the liquid-liquid extraction step until the organic phase was colorless. Extracts were combined, and diethyl ether was eliminated by rotoevaporation. This derivatization process, when applied to algae extracts, was named "E-MEDPA Derivatization Process".

All extracts (both chemically and enzymatically obtained) were independently analyzed by high performance liquid chromatography (HPLC) in a device equipped with a photodiode array detector and a C18 column (250 mm x 4.6 mm x 5 µm). The system was previously equilibrated with methanol (solvent A) and water (solvent B) at 1 ml/min 86% A constant flow. 20 µl samples were injected at 86% A during 10 min, raised to 100% A during 10 min, held 40 min. Detection wavelength was set at 450 nm.

The remaining extract from the chemical derivatization process contained up to 281 mg g⁻¹ of fucoxanthin, 132 mg g⁻¹ of fucoxanthinol, and other carotenoids derived from the saponification reaction such as amaurociaxanthin A (137 mg g⁻¹) or isofucoxanthinol (72 mg g⁻¹), with a final recovery up to 70% of the initial fucoxanthin. Other naturally occurring carotenoids can also be in the extract, such as diadinoxanthin, diazoxanthin or β-carotene. Results and experimental conditions are summarized in Tables 1 and 10.

The remaining extract from the enzymatic (pig liver lipase) derivatization process contained up to 722 mg g⁻¹ of fucoxanthin and 242 mg g⁻¹ of fucoxanthinol, with a final recovery up to 97% of the initial fucoxanthin. Results and experimental conditions are summarized in Tables 1 and 10.

**Table 1**

| **Results and experimental conditions of chemical and enzymatic derivatization of pure fucoxanthin** | | | | | | |
|---|---|---|---|---|---|---|
| Hydrolysis | Solvent | Reaction time | Fucoxanthin | Fucoxanthinol | Amarouciaxanthin A | Isofucoxanthinol |
| No hydrolysis | - | - | 1000 mg g⁻¹ | 0 mg g⁻¹ | 0 mg g⁻¹ | 0 mg g⁻¹ |
| Chemical (KOH) | Methanol | 60 min | 281 mg g⁻¹ | 132 mg g⁻¹ | 137 mg g⁻¹ | 72 mg g⁻¹ |
| Enzymatic (Lipase) | PBS | 24 h | 722 mg g⁻¹ | 242 mg g⁻¹ | 0 mg g⁻¹ | 0 mg g⁻¹ |

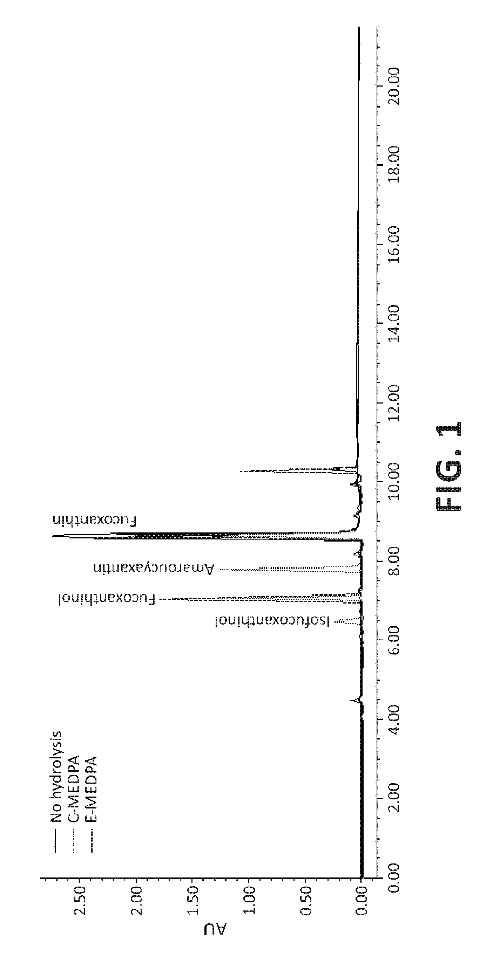

Figure 1 shows an overlaid HPLC chromatogram of the initial fucoxanthin, together with the HPLC chromatograms of the reaction products resulting from the chemical derivatization process (C-MEDPA) and from the enzymatic derivatization process (E-MEDPA).

### EXAMPLE 2

### Haptophyte and Heterokontophyte Microalgae Extracts Derivatization

A group of haptophyte and heterokontophyte strains were selected to evaluate the efficacy of the chemical derivatization process (C-MEDPA) on extracts of such microalgae genus rich in xantophylls, and specifically in fucoxanthin.

Cultures of the *Isochrysis aff. galbana* UTEX LB2307 strain belonging to the class *Prymnesiophyceae; Thalassiosira pseudonana* UTEX LBFD2 and *Navicula incerta* UTEX 2044 belonging both to the class *Bacillariophyceae; Pavlova lutheri* UTEX LB1293 belonging to the class *Pavlophyceae;* and *Ochromonas* sp. UTEX LB275 strain belonging to the class *Crysophyceae,* were sourced from The Culture Collection of Algae at the University of Texas at Austin.

Once the cultures entered in the growth stationary phase, biomass was centrifuged and lyophilized. Initial extracts were obtained by ethanol addition to the dry biomass in 3:1 ratio (three parts liters of ethanol for each kg of dry biomass), repeating this step until the extract was colorless. Ethanolic extracts were filtered, in order to eliminate possible residual dead cells. Obtained extracts were rotovapored to dryness. The soft extract obtained was dissolved in the minimum possible amount of diethyl ether and processed as described in Example 1 following the C-MEDPA derivatization process but replacing the derivatization agent (10 g l⁻¹ of potassium hydroxide) with 6 g l⁻¹ of potassium hydroxide.

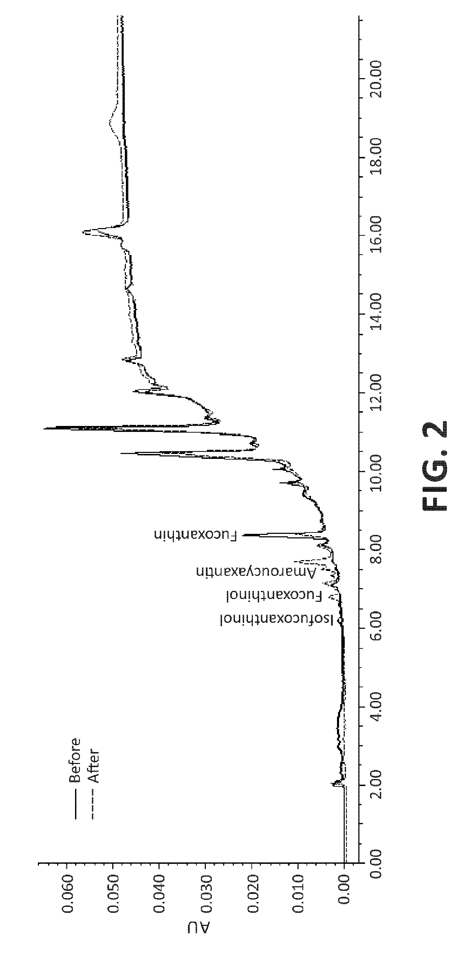

All extracts, before and after the C-MEDPA derivatization process, were analyzed by HPLC as described in Example 1. Concentration of fucoxanthin and fucoxanthin derivatives in the remaining extracts are shown in Table 2 (in mg g⁻¹) and Table 3 (in % of reaction products). Experimental conditions are summarized also in Table 4. As illustrative, Figure 2 shows an overlaid HPLC chromatogram of the *P. lutheri* extract before and after the C-MEDPA derivatization process.

**Table 2**

| **Concentration of carotenoids in microalgae extracts and in C-MEDPA derivatized microalgae extracts (mg g⁻¹)** | | | | | |
|---|---|---|---|---|---|
| | Before derivatization | After derivatization | | | |
| Species | Fucoxanthin | Fucoxanthin | Fucoxanthinol | Amarouciaxanthin A | Isofucoxanthinol |
| *I. aff. galbana* | 13.3 | 2.46 | 2.89 | 3.29 | 1.80 |
| *T. pseudonana* | 9.34 | 0.91 | 1.71 | 2.53 | 5.25 |
| *P. lutheri* | 9.50 | 6.18 | 2.55 | 0.56 | 3.36 |
| *N. incerta* | 2.94 | 0.59 | 1.85 | 2.53 | 0.88 |
| *Ochromonas* sp. | 4.17 | 0.41 | 0.77 | 1.02 | 1.62 |

**Table 3**

| **Concentration of carotenoids in microalgae extracts (% of reaction products)** | | | | | |
|---|---|---|---|---|---|
| | Before derivatization | After derivatization | | | |
| Species | Fucoxanthin | Fucoxanthin | Fucoxanthinol | Amarouciaxanthin A | Isofucoxanthinol |
| *I. aff. galbana* | 100 | 23.6 | 27.7 | 31.5 | 17.2 |
| *T. pseudonana* | 100 | 8.71 | 16.4 | 24.3 | 50.5 |
| *P. lutheri* | 100 | 48.9 | 20.2 | 4.40 | 26.5 |
| *N. incerta* | 100 | 10.1 | 31.5 | 43.2 | 15.1 |
| *Ochromonas* sp. | 100 | 10.8 | 20.1 | 26.7 | 42.4 |

**Table 4**

| **Experimental conditions of the chemical derivatization process (C-MEDPA) applied to Haptophyte and Heterokontophyte Microalgae Extracts** | | | | |
|---|---|---|---|---|
| Species | Culture | Hydrolysis | Solvent | Reaction time |
| *I. aff. galbana* | 2 l Erlenmeyer flask | 6 g l⁻¹ KOH | Methanol | 60 min |
| *T. pseudonana* | 2 l Erlenmeyer flask | 6 g l⁻¹ KOH | Methanol | 60 min |
| *P. lutheri* | 2 l Erlenmeyer flask | 6 g l⁻¹KOH | Methanol | 60 min |
| *N. incerta* | 2 l Erlenmeyer flask | 6 g l⁻¹ KOH | Methanol | 60 min |
| *Ochromonas* sp. | 2 l Erlenmeyer flask | 6 g l⁻¹KOH | Methanol | 60 min |

### EXAMPLE 3

### Scale-up of the C-MEDPA derivatization process for obtaining novel bioactive extracts from I. galbana

For the scale-up of the C-MEDPA derivatization process applied to *I. galbana* cultures, a specific mutant of *I. galbana* UTEX LB2307 strain, named GAT-6007.00.002, was selected. *I. galbana* GAT-6007.00.002 was obtained through a directed evolution process to high exposure to UV light of UTEX LB2307 strain cultures (as described by Bougaran et al., 2012. Enhancement of Neutral Lipid Productivity in the Microalgae Isochrysis Affinis Galbana (T-ISO) by a Mutation-Selection procedure. Biotechnol. Bioengin. 109(11):2737:45) and mutants were sorted by flow cytometry for survival and significant high xanthophills contents (as described by Toepel et al., 2004. Cytometry, 4th Edition. New Developments. Methods in Cell Biology Vol. 15. Chapter 15). GAT-6007.00.002 is a selected variation of UTEX LB2307 strain that behaves mainly as the wild-type with a significant enhancement (around 10% increase) of fucoxanthin accumulation at the tested growth conditions.

Cultures of *I. galbana* GAT-6007.00.002 were carried out in f/2 medium (Guillard and Ryther, 1962. Studies of marine planktonic diatoms. I. Cyclotella nana Hustedt and Detonula confervacea Cleve. Can. J. Microbiol. 8: 229-239) in 50 1 air-lift column photobioreactors, with a light intensity of 100 µmol photons m⁻² s⁻¹, temperature of 25°C and bubbling CO₂ enriched air at 1 1 min⁻¹. Once the culture entered in the stationary phase of growth, biomass was centrifuged and lyophilized. Initial extracts of fucoxanthin and C-MEDPA derivatized extracts were obtained as described in Example 2.

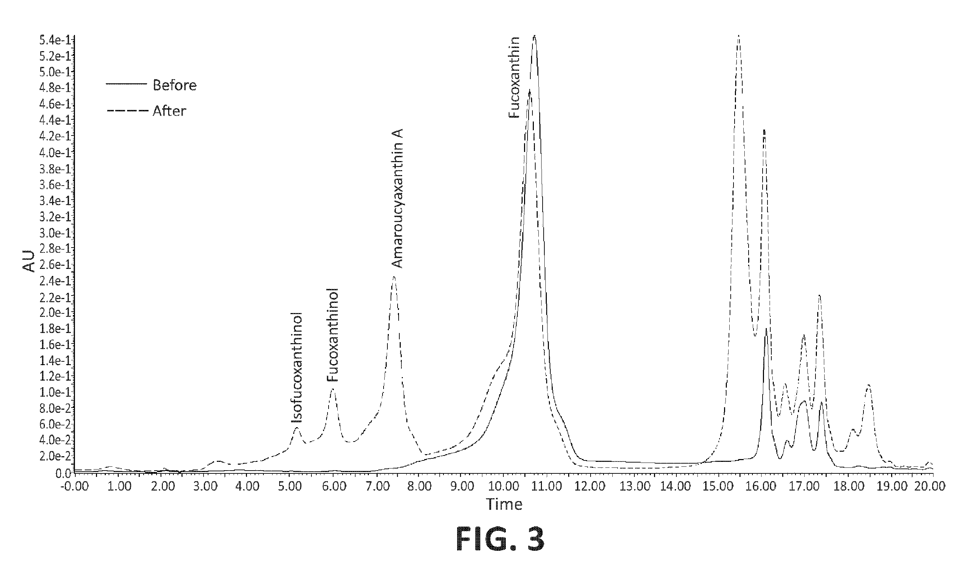

Both extracts, before and after the C-MEDPA derivatization process, were characterized by HPLC as described in Example 1 and chromatograms showed in Figure 3. The C-MEDPA derivatized extract contained up to 50 mg g⁻¹ of fucoxanthin, 15 mg g⁻¹ of fucoxanthinol, among other carotenoids derived from the saponification reaction such as amaurociaxanthin A (20 mg g⁻¹) or isofucoxanthinol (10 mg g⁻¹), with a final recovery up to 95% of the initial fucoxanthin. Other naturally occurring carotenoids can also be in the extract, such as diadinoxanthin, diazoxanthin or β-carotene. Results and experimental conditions are summarized in Table 5.

**Table 5**

| **Results and experimental conditions of the C-MEDPA derivatization process applied to *I. galbana* extracts** | | | | | | | | |
|---|---|---|---|---|---|---|---|---|
| Species | Culture | Hydrolysis | Solvent | Reaction time | Fucoxanthin | Fucoxanthinol | Amarouciaxanthin A | Isofucoxanthinol |
| *I. galbana* | 50 l PBR | No hydrolysis | Methanol | - | 100 mg g⁻¹ | 0 mg g⁻¹ | 0 mg g⁻¹ | 0 mg g⁻¹ |
| *I. galbana* | 50 l PBR | 6 g l⁻¹ KOH | Methanol | 60 min | 50 mg g⁻¹ | 15 mg g⁻¹ | 20 mg g⁻¹ | 10 mg g⁻¹ |

Figure 3 shows the HPLC chromatogram of the *I. galbana* extract before (A) and after (B) the C-MEDPA derivatization process.

### EXAMPLE 4

### Scale-up of the C-MEDPA derivatization process for obtaining novel bioactive extracts from Thalassiosira pseudonana UTEX LBFD2

For the scale-up of the C-MEDPA derivatization process applied to *Thalassiosira pseudonana* cultures, the strain of Example 2 was chosen, i.e., *T. pseudonana* UTEX LBFD2. The production of microalgal biomass was carried as described in Example 3 and the C-MEDPA derivatized extract as in Example 2 but replacing the derivatization agent (6 g l⁻¹ of potassium hydroxide) with 2 g l⁻¹ of potassium hydroxide. Characterization of the obtained extracts was performed following the HPLC protocol described in Example 1.

The C-MEDPA derivatized extract contained 80 mg g⁻¹ of fucoxanthin, 7 mg g⁻¹ of fucoxanthinol, among other carotenoids derived from the saponification reaction such as amaurociaxanthin A (7 mg g⁻¹) or isofucoxanthinol (1 mg g⁻¹). Other naturally occurring carotenoids can also be in the extract, such as diadinoxanthin, diazoxanthin or β-carotene. Results and experimental conditions are summarized in Table 6.

**Table 6**

| **Results and experimental conditions of the C-MEDPA derivatization process applied to *T*. *pseudonana* extracts** | | | | | | | | |
|---|---|---|---|---|---|---|---|---|
| Species | Culture | Hydrolysis | Solvent | Reaction time | Fucoxanthin | Fucoxanthinol | Amarouciaxanthin A | Isofucoxanthinol |
| *T. pseudonana* | 50 l PBR | No hydrolysis | Methanol | - | 93 mg g⁻¹ | 0 mg g⁻¹ | 0 mg g⁻¹ | 0 mg g⁻¹ |
| *T. pseudonana* | 50 l PBR | 2 g l⁻¹ KOH | Methanol | 60 min | 80 mg g⁻¹ | 7 mg g⁻¹ | 7 mg g⁻¹ | 1 mg g⁻¹ |

### EXAMPLE 5

### Scale-up of the E-MEDPA derivatization process for obtaining novel bioactive extracts from I. galbana

A second fraction of the lyophilized biomass from *I. galbana* GAT-6007.00.002 obtained as described in Example 3, is processed to fucoxanthin enriched extracts by ethanol extraction according to the protocols described in Example 2 (in 3:1 proportion ethanol:lyophilized biomass). This first fucoxanthin enriched extract was then derivatized following the enzymatic strategy tested in Example 1 and identified as E-MEDPA derivatization process. Thus, 100 mg of taurocholic acid per gram of extract were added to the fucoxanthin enriched extract and rotoevaporated to dryness. The remaining dried extract was dissolved in aqueous phosphate buffer (PBS) pH 7 containing 6 grams of pig liver lipase (100-400 units/mg, Sigma-Aldrich) per gram of biomass, or alternatively, in another approach, in aqueous phosphate buffer (PBS) pH 7 containing 50 units of cholesterol esterase from *Pseudomonas fluorescens* per gram of biomass (Sigma-Aldrich), and incubated at 37°C for 24 hours. Carotenoids were extracted with subsequent 1:1 volumes of diethyl ether, repeating the liquid-liquid extraction step until the organic phase was colorless. Extracts were combined, and diethyl ether was eliminated by rotoevaporation. The obtained E-MEDPA derivatized extracts contained 65 mg g⁻¹ of fucoxanthin/35 mg g⁻¹ of fucoxanthinol and 13 mg g⁻¹ of fucoxanthin/86 mg g⁻¹ of fucoxanthinol, respectively, from pig liver lipase and cholesterol esterase. Other naturally occurring carotenoids, such as diadinoxanthin, diazoxanthin or β-carotene were also present in the composition at lower amounts. Results and experimental conditions are summarized in Table 7.

**Table 7**

| **Results and experimental conditions of the E-MEDPA derivatization process applied to *I. galbana* extracts** | | | | | | | | |
|---|---|---|---|---|---|---|---|---|
| Species | Culture | Hydrolysis | Solvent | Reaction time | Fucoxanthin | Fucoxanthinol | Amarouciaxanthin A | Isofucoxanthinol |
| *I. galbana* | 50 l PBR | No hydrolysis | Methanol | - | 100 mg g⁻¹ | 0 mg g⁻¹ | 0 mg g⁻¹ | 0 mg g⁻¹ |
| *I. galbana* | 50 l PBR | Lipase | PBS | 24 h | 65 mg g⁻¹ | 35 mg g⁻¹ | 0 mg g⁻¹ | 0 mg g⁻¹ |
| *I. galbana* | 50 l PBR | Esterase | PBS | 24 h | 13 mg g⁻¹ | 86 mg g⁻¹ | 0 mg g⁻¹ | 0 mg g⁻¹ |

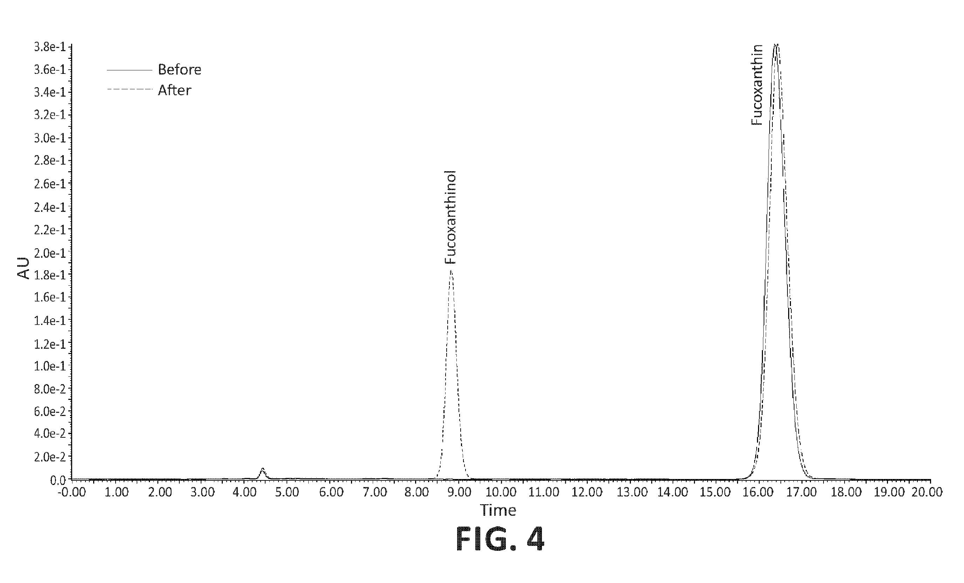

Figure 4 shows the profiles of a cholesterol esterase enzymatically hydrolyzed fucoxanthin-fucoxanthinol extract obtained compared to an initial fucoxanthin enriched extract.

### EXAMPLE 6

### Scale-up of the C-MEDPA derivatization process for obtaining novel bioactive extracts from I.galbana - variation (I)

A third fraction of the lyophilized biomass from *I. galbana* GAT-6007.00.002 (Example 5) was processed to render fucoxanthin enriched extracts by ethanol extraction according to the protocols described in Example 2 (in 3:1 proportion ethanol: lyophilized biomass). This first fucoxanthin enriched extract was then derivatized following a variation of the chemical derivatization process tested in Example 1 and identified as the C-MEDPA derivatization process; thus, once the solid residue was dissolved in the minimum possible amount of solvent, the same volume of ethanol containing 4 g l⁻¹ of potassium hydroxide was added to the ether diethylic extract. The C-MEDPA derivatization process was carried out under nitrogen atmosphere, in the dark and at 0°C, during 10 minutes.

To stop the reaction, the same protocol described in Example 3 was used. Finally, diethyl ether was eliminated by rotoevaporation. The remaining derivatized extract contained 30 mg g⁻¹ of fucoxanthin, 3 mg g⁻¹ of fucoxanthinol, among other carotenoids derived from the saponification reaction such as amaurociaxanthin A (13 mg g⁻¹) or isofucoxanthinol (1 mg g⁻¹). Other naturally occurring carotenoids can also be in the extract, such as diadinoxanthin, diazoxanthin or β-carotene. Results and experimental conditions are summarized in Table 8.

**Table 8**

| **Results and experimental conditions of C-MEDPA derivatization process applied to *I. galbana* extracts - Variation (I)** | | | | | | | | |
|---|---|---|---|---|---|---|---|---|
| Species | Culture | Hydrolysis | Solvent | Reaction time | Fucoxanthin | Fucoxanthinol | Amarouciaxanthin A | Isofucoxanthinol |
| *I. galbana* | 50 l PBR | No hydrolysis | Methanol | - | 100 mg g⁻¹ | 0 mg g⁻¹ | 0 mg g⁻¹ | 0 mg g⁻¹ |
| *I. galbana* | 50 l PBR | KOH 4 g l⁻¹ | Ethanol | 60 min | 30 mg g⁻¹ | 3 mg g⁻¹ | 13 mg g⁻¹ | 1 mg g⁻¹ |

### EXAMPLE 7

### Scale-up of the C-MEDPA derivatization process for obtaining novel bioactive extracts from I. galbana - variation (II)

A fourth fraction of the lyophilized biomass from *I. galbana* GAT-6007.00.002 (Example 5) was processed to fucoxanthin enriched extracts by ethanol extraction according to the protocols described in Example 2 (in 3:1 proportion ethanol: lyophilized biomass). This first fucoxanthin enriched extract was then derivatized following a variation of the chemical derivatization process tested firstly in Example 1 and identified as the C-MEDPA derivatization process; thus, once the solid residue was dissolved in the minimum possible amount of solvent, 0.1 volumes of 0.1 M lithium *tert-*butoxide in THF was added. The C-MEDPA derivatization process was carried out under nitrogen atmosphere, in the dark and at 0°C, during 10 minutes.

To stop the reaction, the same protocol described in Example 3 was used. Finally, diethyl ether was eliminated by rotoevaporation. The remaining derivatized extract contained 20 mg g⁻¹ of fucoxanthin, 10 mg g⁻¹ of fucoxanthinol, among other carotenoids derived from the saponification reaction such as amaurociaxanthin A (10 mg g⁻¹) or isofucoxanthinol (5 mg g⁻¹). Other naturally occurring carotenoids can also be in the extract, such as diadinoxanthin, diazoxanthin or β-carotene. Results and experimental conditions are summarized in Table 9.

**Table 9**

| **Results and experimental conditions of C-MEDPA derivatization process applied to *I. galbana* extracts - Variation (II)** | | | | | | | | |
|---|---|---|---|---|---|---|---|---|
| Species | Culture | Hydrolysis | Solvent | Reaction time | Fucoxanthin | Fucoxanthinol | Amarouciaxanthin A | Isofucoxanthinol |
| *I. galbana* | 50 l PBR | No hydrolysis | MeOH | - | 100 mg g⁻¹ | 0 mg g⁻¹ | 0 mg g⁻¹ | 0 mg g⁻¹ |
| *I. galbana* | 50 l PBR | Li *tert-*ButO | THF | 10min | 20 mg g⁻¹ | 10 mg g⁻¹ | 10 mg g⁻¹ | 5 mg g⁻¹ |

### EXAMPLE 8

### Scale-up of the C-MEDPA derivatization process for obtaining novel bioactive extracts from I. galbana - variation (III)

A fifth fraction of the lyophilized biomass from *I. galbana* GAT-6007.00.002 (Example 5) was processed to fucoxanthin enriched extracts by ethanol extraction according to the protocols described in Example 2 (in 3:1 proportion ethanol: lyophilized biomass). This fucoxanthin enriched extract was then derivatized following a variation of the chemical derivatization process tested in Example 1 and identified as the C-MEDPA derivatization process; thus, once the solid residue was dissolved in the minimum possible amount of solvent, 0.5 volumes of 30% aqueous NH₄OH was added. The C-MEDPA derivatization process was carried out under nitrogen atmosphere, in the dark and at 0°C, during 60 minutes.

To stop the reaction, the same protocol described in Example 3 was used. Finally, diethyl ether was eliminated by rotoevaporation. The remaining derivatized extract contained 80 mg g⁻¹ of fucoxanthin, 3 mg g⁻¹ of fucoxanthinol, among other carotenoids derived from the saponification reaction such as amaurociaxanthin A (3 mg g⁻¹). Other naturally occurring carotenoids can also be in the extract, such as diadinoxanthin, diazoxanthin or β-carotene. Results and experimental conditions are summarized in Table 10.

**Table 10**

| **Results and experimental conditions of C-MEDPA derivatization process of *I. galbana* extracts - Variation (III)** | | | | | | | | |
|---|---|---|---|---|---|---|---|---|
| Species | Culture | Hydrolysis | Solvent | Reaction time | Fucoxanthin | Fucoxanthinol | Amarouciaxanthin A | Isofucoxanthinol |
| *I. galbana* | 50 l PBR | No hydrolysis | MeOH | - | 100 mg g⁻¹ | 0 mg g⁻¹ | 0 mg g⁻¹ | 0 mg g⁻¹ |
| *I. galbana* | 50 l PBR | NH₄OH | MeOH | 60 min | 80 mg g⁻¹ | 3 mg g⁻¹ | 3 mg g⁻¹ | 0 mg g⁻¹ |

**Table 11**

| **Summary of the experimental conditions for C-MEDPA and E-MEDPA derivatization processes** | | | | | | | | | |
|---|---|---|---|---|---|---|---|---|---|
| | | | | | | % of pigment in extract | | | |
| Exampl e | Species | Culture | Derivatization Agent | Solvent | Rection time | Fucoxanthi n | Fucoxanthino 1 | Amarouciaxanthi n A | Isofucoxanthinol |
| 1 | Pure fucoxanthin | - | 10 g/l KOH | Methanol | 60 min | 28.1 | 13.2 | 13.7 | 7.2 |
| 1 | Pure fucoxanthin | **-** | Lipase | PBS | 24 hours | 72.2 | 24.2 | 0.00 | 0.00 |
| 2 | *Isochrysis galbana* | 2 l Erlenmeyer flask | 6 g/l KOH | Methanol | 60 min | 0.25 | 0.29 | 0.33 | 0.18 |
| **2** | *Thalassiosira pseudonana* | 2 l Erlenmeyer flask | 6 g/l KOH | Methanol | 60 min | 0.09 | 0.17 | 0.25 | 0.53 |
| 2 | *Pavlova lutheri* | 2 l Erlenmeyer flask | 6 g/l KOH | Methanol | 60 min | 0.62 | 0.26 | 0.06 | 0.34 |
| 2 | *Navicula incerta* | 2 1 Erlenmeyer flask | 6 g/l KOH | Methanol | 60 min | 0.06 | 0.19 | 0.25 | 0.09 |
| 2 | *Ochromonas sp.* | 2 l Erlenmeyer flask | 6 g/l KOH | Methanol | 60 min | 0.04 | 0.08 | 0.10 | 0.16 |
| 3 | *Isochrysis galbana* | 50 l photobioreactor | No | - | - | 9.80 | 0.00 | 0.00 | 0.00 |
| 3 | *Isochrysis galbana* | 50 l photobioreactor | 6 g/l KOH | Methanol | 60 min | 5.00 | 1.50 | 2.00 | 1.00 |
| 4 | *Thalassiosira pseudonana* | 50 l photobioreactor | 2 g/l KOH | Methanol | 60 min | 8.00 | 0.70 | 0.70 | 0.10 |
| **5** | *Isochrysis galbana* | 50 l photobioreactor | Lipase | PBS | 24 hours | 6.50 | 3.50 | 0.00 | 0.00 |
| **5** | *Isochrysis galbana* | 50 l photobioreactor | Esterase | PBS | 24 hours | 1.30 | 8.60 | 0.00 | 0.00 |
| 6 | *Isochrysis galbana* | 50 l photobioreactor | 4 g/l KOH | Ethanol | 10 min | 3.00 | 0.30 | 1.30 | 1.00 |
| 7 | *Isochrysis galbana* | 50 l photobioreactor | 0.1M lithium tert-butoxide | THF | 10 min | 2.00 | 1.00 | 1.00 | 0.50 |
| 8 | *Isochrysis galbana* | 50 l photobioreactor | 30% aqueous NH₄OH | Methanol | 60 min | 8.00 | 0.30 | 0.30 | 0.00 |

### EXAMPLE 9

### Antiinflammatory effect of the C-MEDPA and E-MEDPA derivatized extracts compared to fucoxanthin enriched extracts of I. galbana

This Example was performed in order to evaluate the anti-inflammatory effect of some representative algal extracts obtained by treatment with the C-MEDPA or with the E-MEDPA derivatization processes. To that end, the *I. galbana* C-MEDPA and E-MEDPA extracts of Examples 3, 5 and 6 were tested.

The *I. galbana* C-MEDPA and E-MEDPA extracts of Examples 3, 5 and 6 have the particulars shown in Table 12.

**Table 12**

| ***I. galbana* C-MEDPA and E-MEDPA extracts** | | | | | | |
|---|---|---|---|---|---|---|
| | | | % of pigment in extract | | | |
| Example | Species | Derivatization Agent | Fucoxanthin | Fucoxanthinol | Amaroucia xanthin A | Isofucoxanthinol |
| **3** | *Isochrysis galbana* | No | 98.0 | 0.00 | 0.00 | 0.00 |
| **3** | *Isochrysis galbana* | 6g/l KOH | 50.0 | 15.0 | 20.0 | 10.0 |
| 6 | *Isochrysis galbana* | 4g/lKOH | 30.0 | 3.00 | 13.0 | 10.0 |
| **5** | *Isochrysis galbana* | Lipase | 65.0 | 35.0 | 0.00 | 0.00 |

The extracts of Table 12 were diluted in DMSO in order to normalize the total content of fucoxanthins (presented in any of the following forms: fucoxanthin, fucoxanthinol, amarouciaxanthin A and isofucoxanthinol) to 100 mg total fucoxanthins/ml.

Normal human epidermal keratinocyte progenitor cells (HPEKp, Cellntec) were grown in a defined keratinocytes medium, CnT-07 (Cellntec), at 37°C with 5% CO₂ in the humidified incubator. After one passage of expansion, cells were used for the experiment.

Keratinocytes were seeded at a density of 10,000 cells/well in a 96 well plate. After two days of culture, cells were treated with the different *I. galbana* extracts (concentrations as described below in Table 13). 24 hours later cells were challenged with PMA phorbol 12-myristate-13-acetate for 5 hours. Plates were then centrifuged, and the cell culture supernatant harvested. Cytokine [interleukin 8 (IL-8)] expression was then determined by ELISA (Human IL-8 Platinum ELISA Kit; Affymetrix eBioscience). All the conditions were performed in triplicates. Expression levels in the cell culture supernatants were normalized to the cell numbers using Janus B green staining to determine cell number. Results were expressed as percentage using untreated control as 100%.

**Table 13**

| ***I. galbana* C-MEDPA and E-MEDPA extracts** | | |
|---|---|---|
| **Sample** | **Concentration** | |
| Example 3 (Fucoxanthin) | 0.000024% | 0.000012% |
| Example 3 (C-MEDPA) | 0.000024% | 0.000012% |
| Example 6 (C-MEDPA) | 0.000024% | 0.000012% |
| Example 5 (E-MEDPA) | 0.000024% | 0.000012% |

The results are shown in Figure 5 wherein it can be appreciated that the production of IL-8 is considerably repressed in keratinocytes treated with the extracts comprising fucoxanthin and fucoxanthinol obtained in Examples 3 and 6 after chemical treatment with the C-MEDPA derivatization process or in Example 5 after enzymatic treatment with the E-MEDPA derivatization process.

### EXAMPLE 10

### Lipolysis effect of the C-MEDPA derivatized extracts of Thalassiossira vseudonana

This Example was performed in order to preliminarily evaluate the lipolysis effect of a representative algal extract obtained by treatment with the C-MEDPA derivatization process. To that end, the *Thalassiosira pseudonana* C-MEDPA extract of Example 4 was tested [that extract was named in this assay as "Extract-1A"].

Briefly, human subcutaneous preadipocytes were seeded in preadipocyte medium into a 96-well plate at a density of 40.625 cells/cm² and were incubated in a humidified 37°C incubator with 5% CO₂. After 48 hours incubation, cells were induced to differentiate by exchanging with adipocyte differentiation medium and the plates were incubated for 7 days in an incubator. After 7 days, cells were fed with fresh adipocyte maintenance medium and were incubated for another 7 days. After differentiation, cells appeared rounded with large lipid droplets apparent in the cytoplasm indicating mature adipocytes. Cells were treated for 3 hours either in the presence of actives ("Extract-1A" at two concentrations in the final assay volume: 0,000048% and 0,000024%) or in buffer alone as untreated control. Isoproterenol treatment was used as a positive control.

After 3 hours of treatment, cell culture supernatants were analysed for glycerol release using an absorbance-based method. A standard curve generated using glycerol standards were used to calculate glycerol released by each actives. Glycerol release/well was normalized to the cell number using Janus B green to determine cell number. All the conditions were performed in triplicates and the results were depicted in percentage release of glycerol normalized to untreated cell controls (100%).

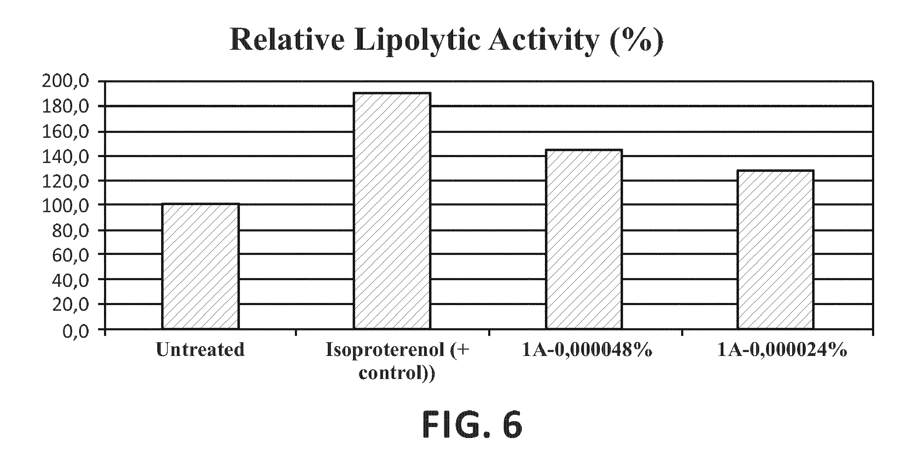

The results (Figure 6) show that:
- at the highest concentration tested (0,000048%), the "Extract-1A" showed an increase in the lipolytic activity of about 45% when compared to the untreated control (i.e., about 50% equivalent with respect to the positive control isoproterenol); and
- at the lowest lower concentration (0,000024%), the "Extract-1A" showed an increase in the lipolytic activity of about 27% when compared to the untreated controls (i.e., about 31% when compared to the positive control isoproterenol).

### EXAMPLE 11

### Analysis of fatty acids in algal extracts of the invention

This Example was carried out in order to analyze the fatty acid (FA) content in some algal extracts provided by this invention obtained after treating an algal culture to the C-MEDPA derivatization process or to the E-MEDPA derivatization process.

Thus, analysis of FA content were performed for the *I. galbana* extracts obtained in Examples 2, 3 and 5 (i.e., subjected to C- or E-MEDPA derivatization process).

Briefly, around 25 mg of freeze-dried sample were weighed in 15 ml polypropylene tubes with screw cap. 0.5 mg of triundecanoin were added as internal standard. Extraction and transesterification of fatty acids was carried out by the addition of 1.25 ml of 0.5M potassium hydroxide in methanol, vigorously vortexed and reacting for 5 minutes at 75°C. Once the sample had cooled down, 2 ml of boron trifluoride were added, and samples were kept again at 75°C for 5 more minutes. Finally, 1 ml of a saturated sodium chloride aqueous solution and 1 ml of hexane were added, and samples were shaken vigorously. Upper phase (hexane) was transferred to chromatographic vials.

Instrumental analysis was carried out by gas chromatography (CG) with flame ionization detector, equipped with a SP-2330 (30 m x 0.25 mm x 0.20 µm, Sigma Aldrich). Two µl were injected in split mode, with the injector set at 270°C, using helium as carrier gas at 1 ml/min and detection temperature of 270°C. Oven initial temperature was set at 150°C, increasing at 4°C/min to 220°C, held 2.5 min. Samples were quantified according to the internal standard method.

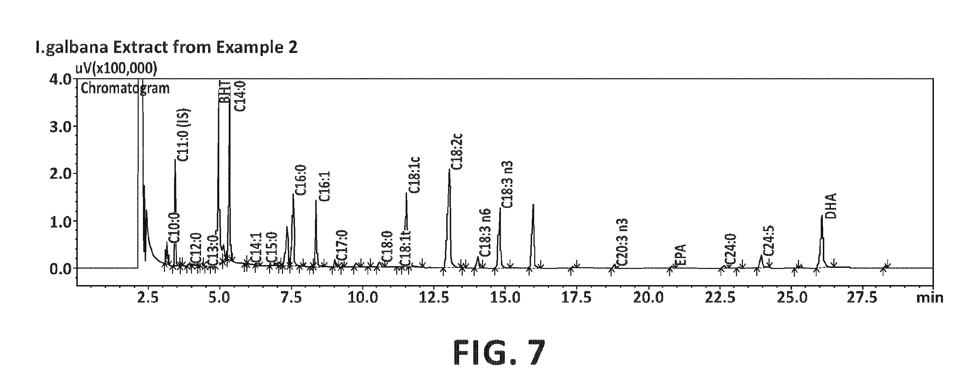

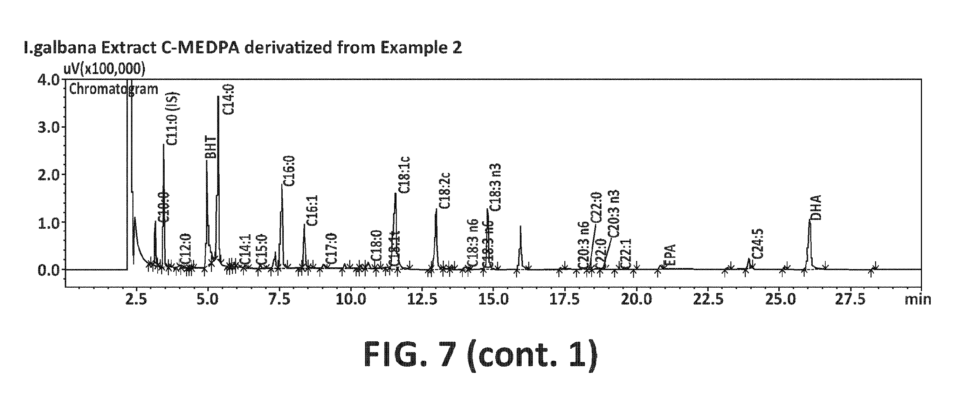

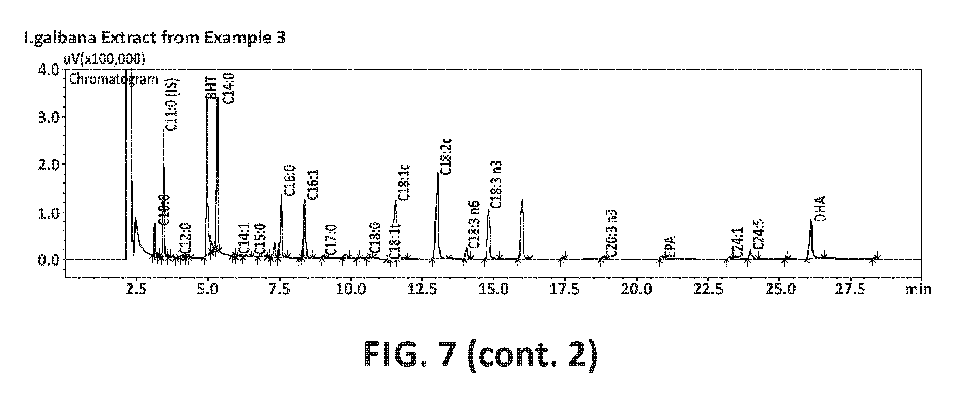

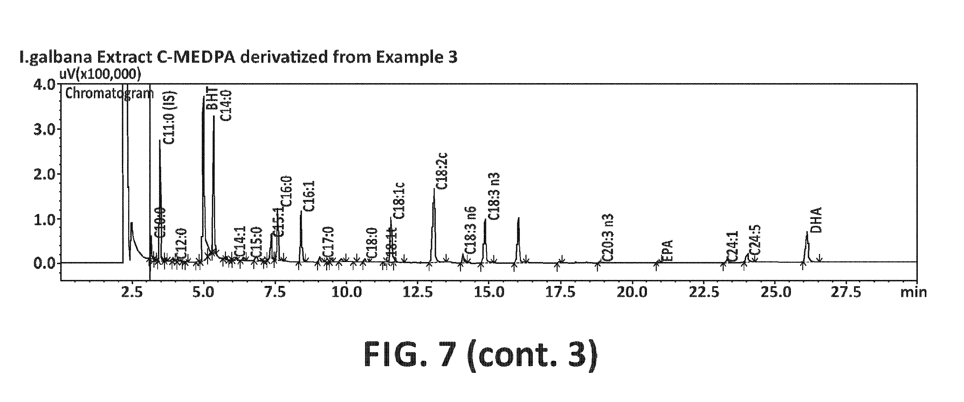

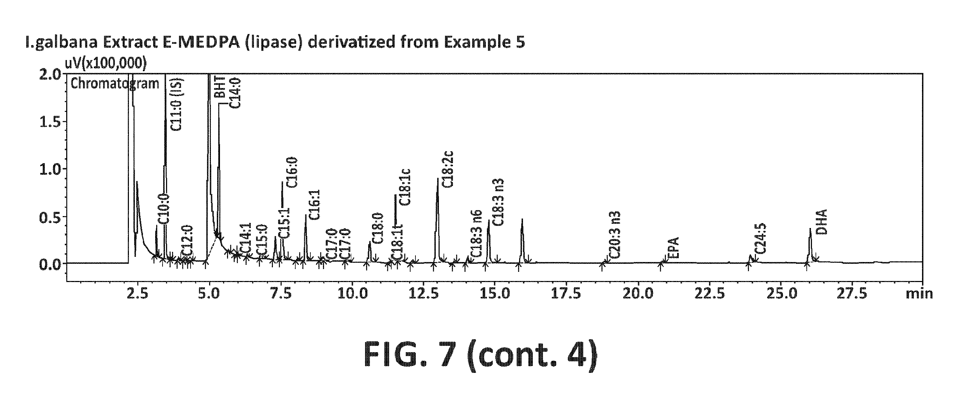

The results are shown in Figure 7 and in Tables 14-16.

### Comments:

1. The C-MEDPA derivatization process as well as the E-MEDPA derivatization process results in no significant effect on the natural FA profiles of crude extracts.
2. The content of PUFAs in the C-MEDPA extracts as well as in the E-MEDPA extracts are higher than 150 mg/g.
3. Total PUFAs (including palmitoleic, oleic, LA, GLA, ALA, ARA) - Specifically the contents on main PUFAs (palmitoleic, oleic, LA, GLA, ALA, and ARA) in the C-MEDPA extracts and E-MEDPA extracts are maintained higher than 130 mg/g.
4. Further, the content of DHA (which has been referenced to increase absorption of fucoxanthin and derivates) in C-MEDPA extracts and E-MEDPA extracts is maintained between 15 and 60 mg / g extract.

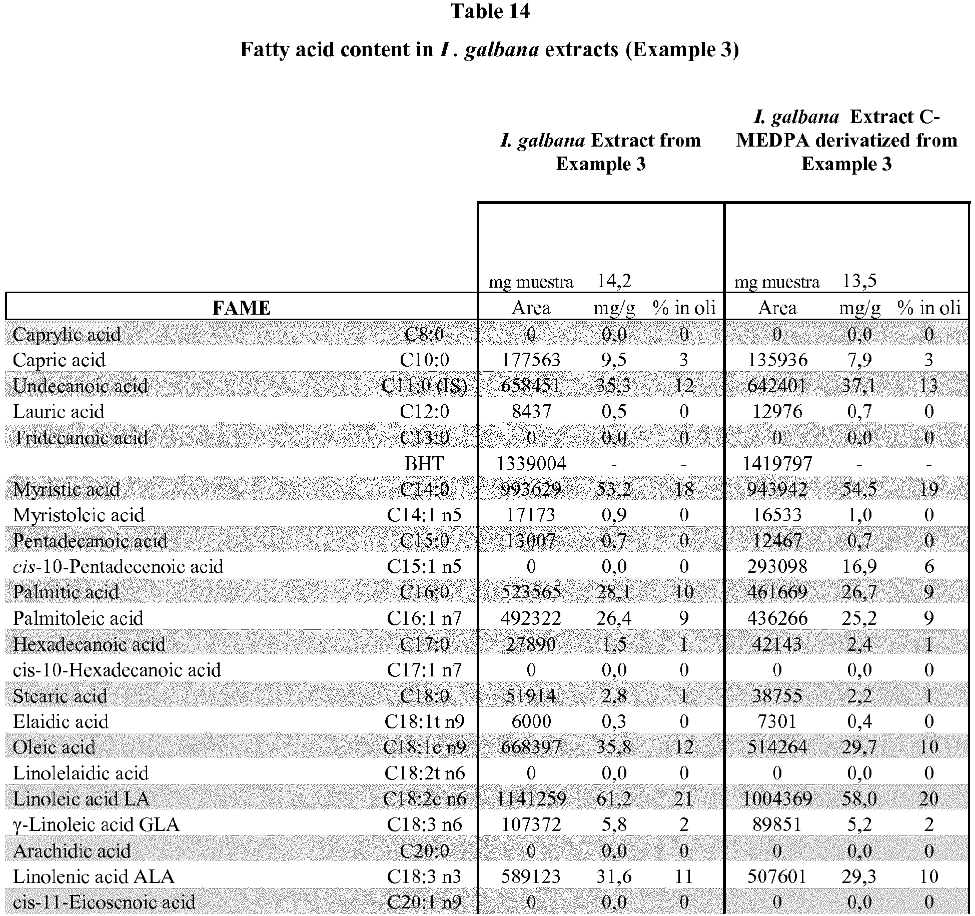

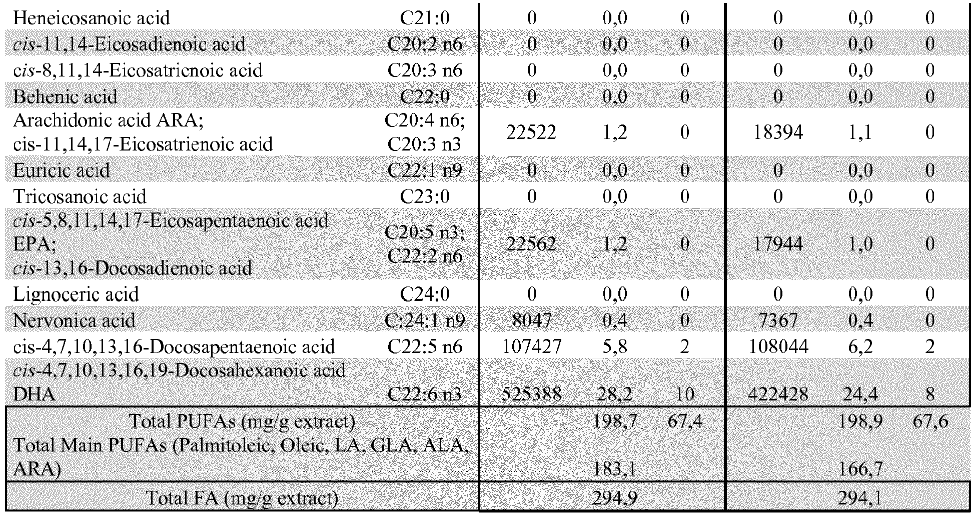

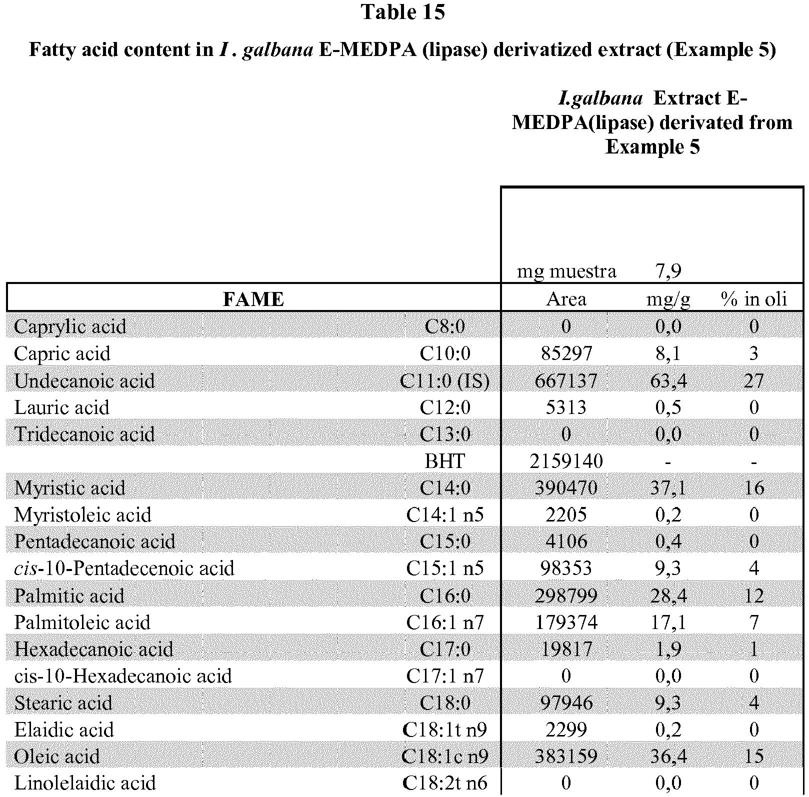

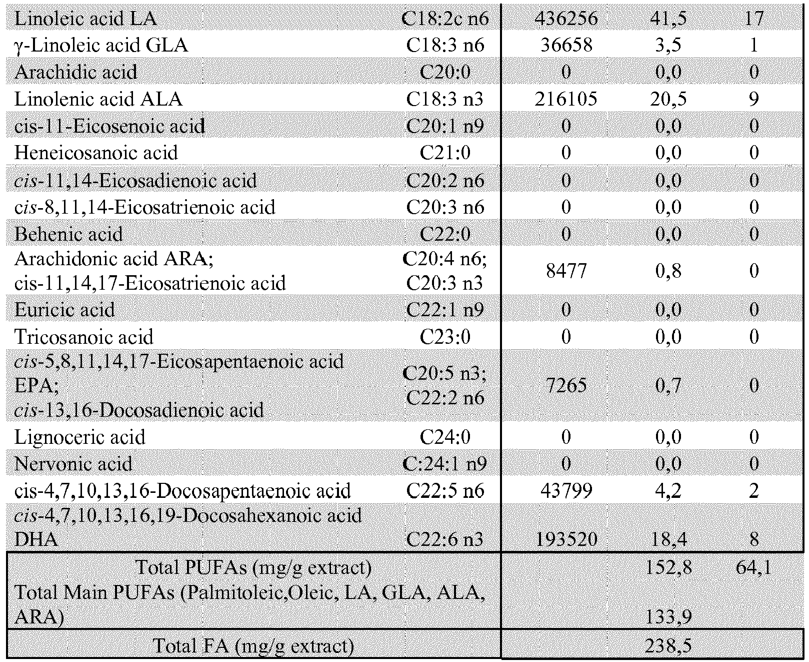

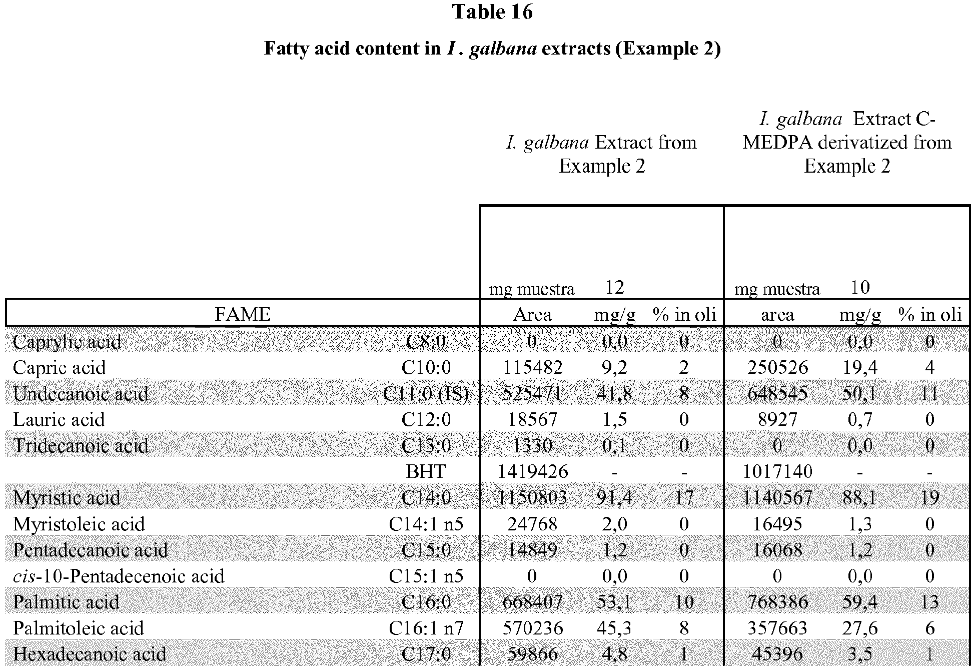

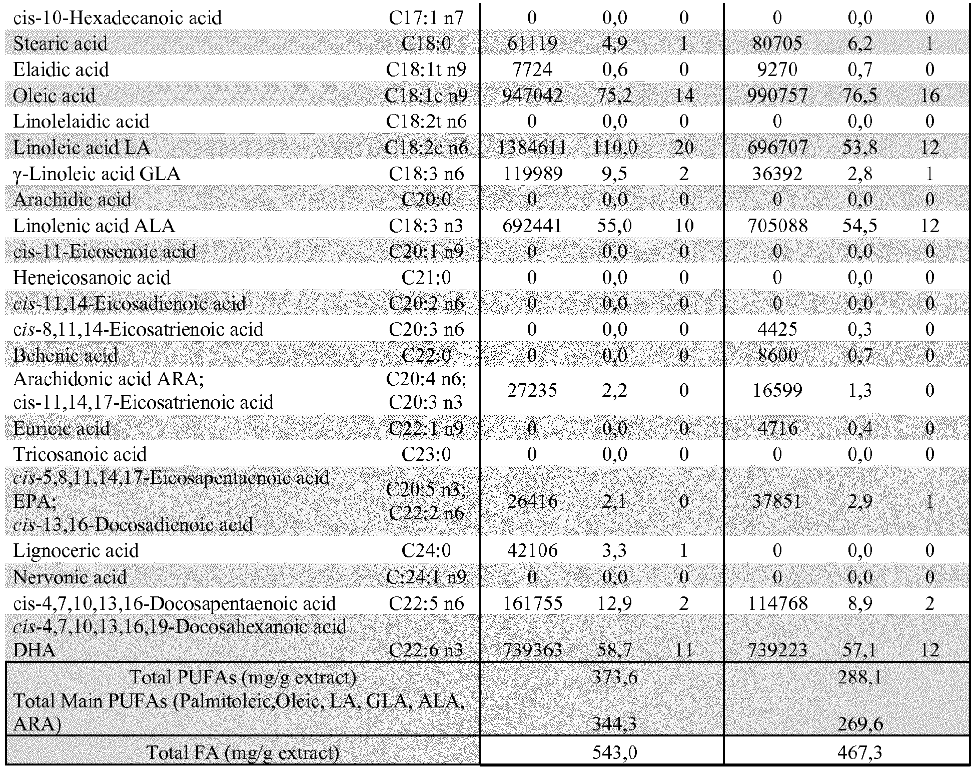

### EXAMPLE 12

### Compositions comprisins an algal extract of the invention

Different compositions for application to the skin in the form of cream emulsions, gels, milks, suspensions, O/W or W/O emulsions, or liposome foams, aqueous or emulsion lotions, sprays or waxy sticks where prepared by using the different algal extracts of the invention, namely, the algal extracts obtained by C-MEDPA or E-MEDPA derivatization processes, listed in Table 11 (identified below as "algal extract").
1) Composition 1: Enriched Oil - cosmetics/cosmeceutics/nutraceutical
   a. Algal extract 1% w/w
   b. Vegetal oil (q.s. ad)
2) Composition 1':
   a. Triglyceride 86.5% w/w
   b. Algal extract 12.5% w/w
   c. Natural Tocopherols 1.0 % w/w
3) Composition 2: Liposomated Extract - cosmetics/cosmeceutics/nutraceutical
   a. Algal extract 1% w/w
   b. Lecithins (20 mg/ml) 9% w/w
   c. Water 90% w/w
4) Composition 3: Emulsion. Cosmetics/Cosmeceutics/Nutraceutical
   a. Algal extract 1% w/w.
   b. Vegetal oil 8,9% w/w
   c. Water 90% w/w
   d. Surfactant (Tween 80®) 0,1 % w/w
5) Composition 4: Cream. Cosmetics-cosmeceutics
   a. Algal extract 5% w/w
   b. Vegetal oil 5% w/w
   c. Water 26,5% w/w
   d. Excipient (xanthan, gelam, chitosan...) 56,4% w/w
   e. Surfactant (Tween 80®) 0,1 % w/w
6) Composition 5: Gel. Cosmeceutic, medical device, wound dressing
   a. Algal extract 5% w/w
   b. Chitosan or alginate or hyaluronic acid (q.s. ad)
7) Composition 6: Capsules. Nutraceuticals
   a. Algal extract 1 % w/w
   b. Fucoidan 10% w/w
   c. Denaturated starch 89% w/w
8) Composition 7: Granulates. Nutraceuticals
   a. Triglyceride 10.0% w/w
   b. Algal extract 2.5% w/w
   c. Cyclodextrin 85.0% w/w
   d. Natural Tocopherols 2.5% w/w

## Claims

1. An algal extract comprising fucoxanthin and fucoxanthinol, and, optionally, amarouciaxanthin A and/or isofucoxanthinol, together with other algal components.

2. Algal extract according to claim 1, which comprises:
- 0.001 % to 90% by weight of fucoxanthin;
- 0.001 % to 90% by weight of fucoxanthinol;
- 0% to 90% by weight of amarouciaxanthin A;
- 0% to 10% by weight of isofucoxanthinol; and
- 1% to 99.998% by weight of other algal components.

3. Algal extract according to claim 1 or 2, which comprises:
- 0.001 % to 90% by weight of fucoxanthin;
- 0.001 % to 90% by weight of fucoxanthinol;
- more than 0% to 90% by weight of amarouciaxanthin A;
- more than 0% to 10% by weight of isofucoxanthinol; and
- 1% to 99.998% by weight of other algal components.

4. Algal extract according to any of claims 1 to 3, which comprises about 1% and about 50% by weight of at least one fatty acid, preferably a polyunsaturated fatty acid (PUFA), more preferably ALA, *cis*-11,14,17-eicosatrienoic acid (C20:3 n3), EPA, DHA, LA, GLA, *cis*-11,14-eicosadienoic acid (C20:2 n6), *cis*-8,11,14-eicosatrienoic acid (C20:3 n6), ARA, *cis*-13,16-docosadienoic acid (C22:2 n6), cis-4,7,10,13,16-docosapentaenoic acid (C22:5 n6), palmitoleic acid (C16:1 n7), oleic acid (C18:1c n9), *cis*-11-eicosenoic acid (C20:1 n9), euricic acid (C22:1 n9), nervonic acid (C:24:1 n9), and combinations thereof.

5. A.process for producing an algal extract comprising fucoxanthin and fucoxanthinol, and, optionally, amarouciaxanthin A and/or isofucoxanthinol, together with other algal components, according to any of claims 1 to 4, which comprises:
a) culturing fucoxanthin producing algae biomass under conditions that allow the production of fucoxanthin; and
b) contacting fucoxanthin with:
b.1) a base under conditions that allow the hydrolysis of at least a portion of fucoxanthin to fucoxanthinol; or, alternatively, with
b.2) an enzyme that catalyzes the conversion of fucoxanthin to fucoxanthinol under conditions that allow the conversion of at least a portion of fucoxanthin to fucoxanthinol.

6. Process according to claim 5, wherein the fucoxanthin producing alga is a macroalgae belonging to the phylum Rhodophyta or Pheophyta, or a microalgae belonging to the phylum Heterokontophyta, Haptophytas or Dinophyta, preferably, a microalga belonging to a class selected from the group consisting of *Rapidophyceae, Bacillariophyceae* (Diatomeas), *Crysophyceae, Pavlophyceae,* and *Prymnesiophyceae,* more preferably, a microalga that belongs to a genus selected from the group consisting of *Isochrysis, Thalassiosira, Navicula, Pavlova, Ochromonas, Phaeodactylum, Odontella, Skeletonema, Chaetoceros, Prymnesium, Nitzschia, Dinobryon, Synura, Chrysochromulina, Ochrosphaera, Cylindrotheca, Chromulina, Mallomonas,* and *Emiliania.*

7. Process according to claim 5 or 6, wherein the base is an inorganic base or an organic base, or wherein the enzyme that catalyzes the conversion of fucoxanthin to fucoxanthinol is an hydrolase, preferably a lipase or a cholesterol esterase.

8. Process according to any of claims 5 to 7, wherein the product resulting from step a) is removed from the culture medium, optionally dried, and subjected to a treatment, comprising:
i) contacting said removed algal biomass containing fucoxanthin with an organic solvent in order to obtain an organic extract comprising fucoxanthin; and, if desired,
ii) drying said organic extract comprising fucoxanthin from step i) to obtain a residue comprising fucoxanthin; and
iii) dispersing said residue comprising fucoxanthin from step ii) in an organic solvent.

9. A process for producing an algal extract comprising fucoxanthin and fucoxanthinol, and, optionally, amarouciaxanthin A and/or isofucoxanthinol, together with other algal components, according to any of claims 1 to 3, which comprises:
a) culturing fucoxanthin producing algae biomass under conditions that allow the production of fucoxanthin;
b) removing the algal biomass comprising fucoxanthin resulting from step a);
c) contacting the algal biomass containing fucoxanthin resulting from step a) with an organic solvent under conditions that allow obtaining an organic extract comprising fucoxanthin; and, if desired, drying said organic extract comprising fucoxanthin to obtain a residue comprising fucoxanthin; and dispersing said residue comprising fucoxanthin in an organic solvent;
d) contacting the product resulting from step c) with fucoxanthin with:
d.1) a base under conditions that allow the hydrolysis of at least a portion of fucoxanthin to fucoxanthinol; o, alternatively, with
d.2) an enzyme that catalyzes the conversion of fucoxanthin to fucoxanthinol under conditions that allow the conversion of at least a portion of fucoxanthin to fucoxanthinol;
e) adding an aqueous saline solution to the product resulting from step d) under conditions that allow the formation of an aqueous phase and an organic phase, wherein the organic phase comprises fucoxanthin and fucoxanthinol; and
f) removing and, if desired, drying the organic phase comprising fucoxanthin and fucoxanthinol, to render an extract comprising fucoxanthin and fucoxanthinol. 1.

10. A process for producing an algal extract comprising at least 37% by weight of fucoxanthin and 25% by weight of fucoxanthinol, and, optionally, amarouciaxanthin A and/or isofucoxanthinol, together with other algal components, which comprises:
a) culturing a fucoxanthin producing microalgal under conditions that allow the production of fucoxanthin;
b) removing and drying said microalgal biomass comprising fucoxanthin;
c) contacting said dry microalgal biomass with ethanol under conditions that allow obtaining an ethanol extract comprising fucoxanthin;
d) drying said ethanol extract comprising fucoxanthin to obtain a solid residue comprising fucoxanthin;
e) dispersing said solid residue comprising fucoxanthin from step d) in diethyl ether;
f) contacting the suspension resulting from step e) with a base in a medium comprising an alcohol;
g) adding an aqueous sodium chloride solution to the product resulting from step f) under conditions that allow the formation of an aqueous phase and an organic phase, wherein the organic phase comprises fucoxanthin and fucoxanthinol;
h) removing the organic phase comprising fucoxanthin and fucoxanthinol;
i) washing said removed organic phase comprising fucoxanthin and fucoxanthinol; and
j) drying said organic phase comprising fucoxanthin and fucoxanthinol to render an extract comprising at least 37% by weight of fucoxanthin and 25% by weight of fucoxanthinol.

11. A composition comprising an algal extract according to any of claims 1 to 4, or obtainable by a process according to any of claims 5 to 10, and a cosmetic, cosmeceutical, food, nutraceutical, or pharmaceutical acceptable vehicle.

12. An algal extract according to any of claims 1 to 4, or obtainable by a process according to any of claims 5 to 10, for use as an adiponectin production accelerator, an antiangiogenic agent, an antiallergic agent, an antiangiogenic agent, an antibacterial agent, an anticancer agent, an antidiabetic agent, an antifungal agent, an antiinflamatory agent, an antimalarial agent, an antineoplastic agent, an antiobesity agent, an antioxidant agent, an antihypertensive agent, a bone protective agent, a cerebrovascular protective agent, a cholesterol lowering agent, an hepatoprotective agent, an ocular protective agent, a neovascularization inhibitor, a skin-protective agent.

13. An algal extract according to any of claims 1 to 4, or obtainable by a process according to any of claims 5 to 10, for use in the prevention and/or treatment of cancer, depression, hyperuricemia, an inflammatory disease, gingivitis, joint/muscle recovery from overexertion, osteoarthritis, psoriasis, rheumatoid arthritis, metabolic syndrome management, osteoporosis, skin pigmentation, virus associated malignancy such as adult T-cell leukemia or Burkitt lymphoma; or for the prevention, amelioration or treatment of damage of mammalian skin, particularly, for inhibiting one or more of the following skin conditions: inflammation, photo-ageing skin damage, uneven pigmentation, wrinkles and sagging skin.

14. A cosmetic, pharmaceutical, nutraceutical or food product comprising an algal extract according to any of claims 1 to 4, or obtainable by a process according to any of claims 5 to 10, comprising between 0.1% and 5% by weight of said algal extract.

15. Cosmetic, pharmaceutical or nutraceutical product according to claim 14, which further comprises a sunscreen agent.
